# EUROPEAN PATENT APPLICATION

(11) **EP 3 091 460 A1**
(43) Date of publication of application: **09.11.2016**
(21) Application number: 15169259.7
(22) Date of filing: 26.05.2015
(51) Int. Cl.: G06F 19/00

(54) **POINT OF CARE TESTING SYSTEM**

(30) Priority: 02.05.2015 EP 15166151; 02.05.2015 EP 15166152
(71) Applicant: F. Hoffmann-La Roche AG, 4070 Basel (CH); Roche Diagnostics GmbH, 68305 Mannheim (DE)
(72) Inventor: MORCILLO MONTEJO, Alejandro, 17310 Lloret de Mar (ES); RUD, Dmytro, 6333 Huenenberg See (CH)
(74) Representative: Gyenge, Zoltán

(57) **Abstract**

Disclosed are a system architecture and a method to enable workflow solutions via remote configuration services for the configuration management of point of care analyzers, wherein according to particular embodiments, the disclosed system and method are configured to enable remote configuration services of point of care analyzers across different platforms.

## Description

### PRIORITY INFORMATION

This application claims the priority of European Patent Applications EP15166151 and EP15166152.

### TECHNICAL FIELD

The invention relates to a point of care testing POCT system and a method for configuration of a point of care testing POCT system.

### BACKGROUND

In vitro diagnostic testing has a major effect on clinical decisions, providing physicians with pivotal information. Particularly, there is great emphasis on providing quick and accurate test results in critical care settings.

One field of diagnostic testing is conducted with large analytical instruments in laboratories. These instruments are operated by operators that are educated to maintain and operate such instruments.

Another field of diagnostic testing is bedside testing or point of care testing POCT. This type of diagnostic testing is performed mainly by nurses or medical staff primarily trained to operate the instruments available at the site of patient care, such as hospitals, emergency departments, intensive care units, primary care setting, medical centers, patient homes, a physician's office, a pharmacy or a site of an emergency .

Often, point of care testing POCT needs to meet clinical and laboratory requirements for short turnaround times in critical care. Rapid determination of time-critical parameters (e.g. blood glucose, cardiac markers, blood gases, etc.) can accelerate decision making in the emergency room, intensive care units or even in the primary care setting.

Major benefits are obtained when the output of a point of care testing POCT device is made available immediately. Results can be shared instantaneously with all members of the medical team, thereby enhancing communication through decreasing turnaround time.

point of care testing POCT has become established worldwide and finds vital roles in public health. Potential operational benefits of point of care testing POCT include: faster decision making, reduced operating times, reduced postoperative care time, reduced emergency room time, reduced number of outpatient clinic visits, reduced number of hospital beds required and overall optimal use of professional time.

While there are many benefits of using point of care testing POCT devices in terms of their convenience, establishing point of care testing POCT is challenging. Some of the biggest challenges relate to engaging health care providers as testing personnel, all the while ensuring adherence to best laboratory practices and regulatory agency standards. Thus point of care testing POCT implementation requires a systematic approach that involves all stakeholders.

Implementation of configuration management workflows for point of care analyzer(s) poses further difficulties due to the high degree of complexity and lack of modularity and adaptability of existing system architectures for point of care testing POCT systems.

Furthermore, the fact that point of care analyzer(s) are by their nature often distributed throughout one or more medical units or facilities, further complicates management of point of care testing POCT systems.

### SUMMARY

Disclosed are a system architecture and a method to enable workflow solutions via remote configuration services for the configuration management of point of care analyzers.

In particular embodiments, the disclosed system and method are configured to enable remote configuration services of point of care analyzers across different platforms.

In particular embodiments, the disclosed system and method provide for convenient assisted workflows that enable efficient replacement of point of care analyzers and enable traceable relocation of point of care analyzers and efficient management of point of care analyzer operator certifications.

### BRIEF DESCRIPTION OF DRAWINGS

Further characteristics and advantages of the disclosed method / device /system will in the following be described in detail by means of the description and by making reference to the drawings. Which show:
- Fig. 1: a simplified diagram of an embodiment of the disclosed point of care testing POCT system;
- Fig. 2: a use case diagram of an embodiment of a disclosed method for configuration of a point of care testing POCT system;
- Fig. 3: an illustrative diagram of an embodiment of the disclosed point of care testing POCT system, including a hardware management server and a remote configuration server;
- Fig. 4: a use case diagram of a further embodiment of a disclosed method for configuration of a point of care testing POCT system including a remote configuration server and a hardware management server;
- Fig. 5A: an illustrative diagram of a further embodiment of the disclosed point of care testing POCT system, the remote configuration server further including an authentication and authorization unit and an adapter unit;
- Fig. 5B: an illustrative diagram of a further embodiment of the disclosed point of care testing POCT system, communicatively connected to a Laboratory Information System LIS and/or Hospital Information system HIS;
- Fig. 6: an illustrative diagram of a further embodiment of the disclosed point of care testing POCT system, the hardware management server including a first hardware management subserver communicatively connected to a first group of one or more point of care analyzer(s) and a second hardware management subserver communicatively connected to a second group of one or more point of care analyzer(s);

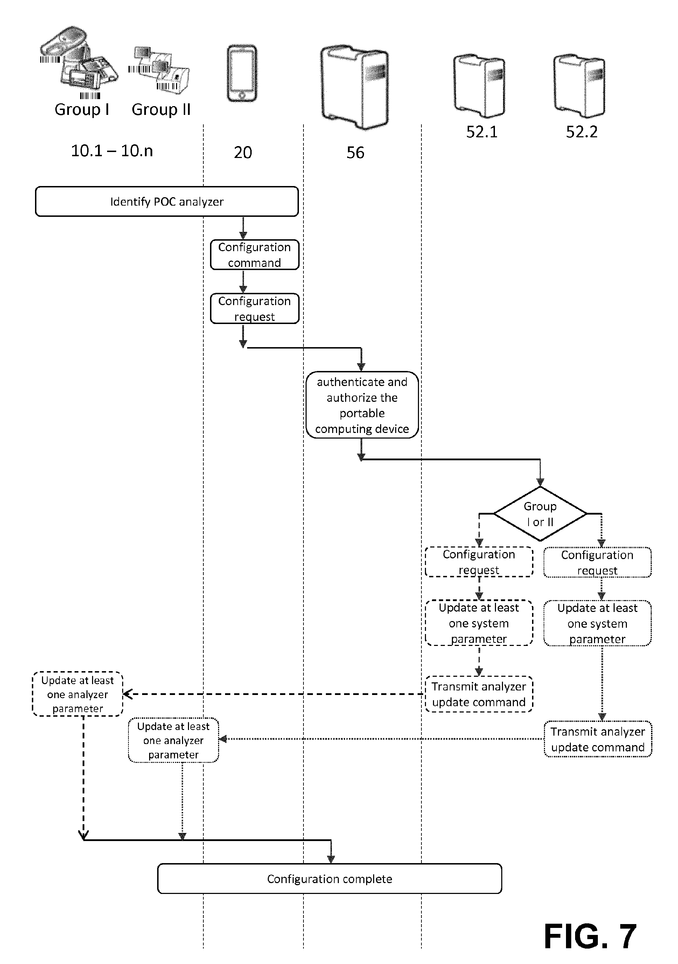

- Fig. 7: a use case diagram of a further embodiment of a disclosed method for configuration of a point of care testing POCT system including a remote configuration server and a first and second hardware management servers;
- Fig. 8A: an illustrative overview of an embodiment of the disclosed communication network;
- Fig. 8B: an illustrative overview of a further embodiment of the disclosed communication network;
- Fig. 8C: an illustrative overview of a further embodiment of the disclosed communication network;
- Fig. 8D: an illustrative overview of an even further embodiment of the disclosed communication network;
- Fig. 9: a use case diagram of an embodiment of a disclosed method for configuration of a point of care testing POCT system, that includes relocation of a point of care analyzer;
- Fig. 10A-10B: illustrative screenshots of a portable computing device; illustrating method steps of a relocation of a point of care analyzer;
- Fig. 11: a use case diagram of an embodiment of a disclosed method for configuration of a point of care testing POCT system, illustrating replacement of a first (defective) point of care analyzer with a second (replacement) point of care analyzer;
- Fig. 12A-12D: illustrative screenshots of a portable computing device; illustrating method steps of for replacement of a first (defective) point of care analyzer with a second (replacement) point of care analyzer;
- Fig. 13: a use case diagram of an embodiment of the disclosed method for configuration of a point of care testing POCT system, illustrating certification configuration corresponding to one or more operator(s) and one or more certification(s); and

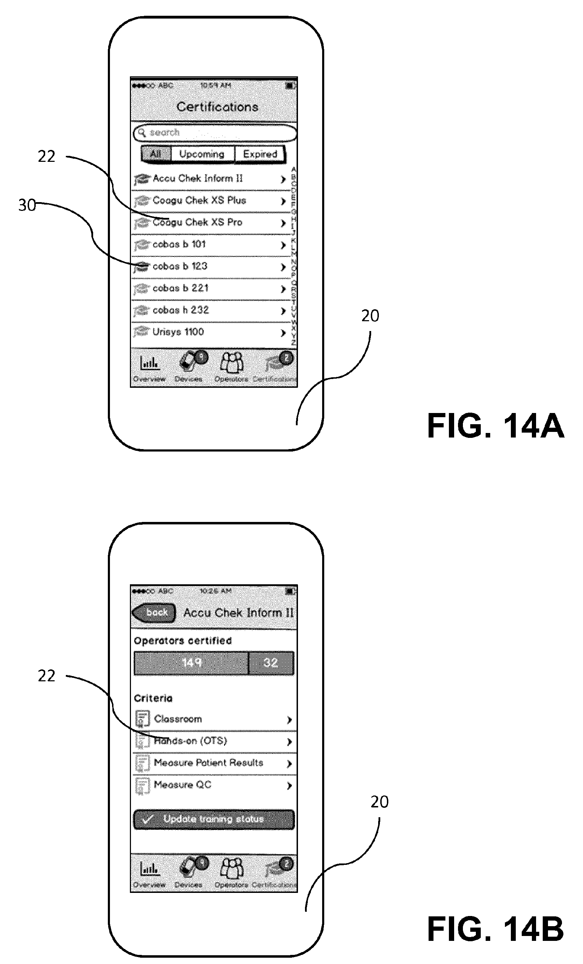

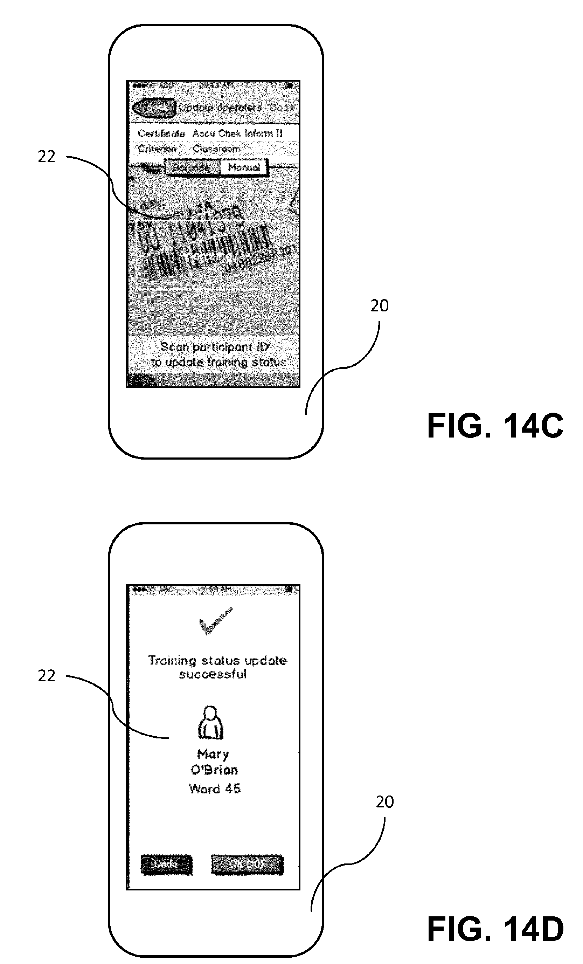

- Fig. 14A-14D: illustrative screenshots of a portable computing device, illustrating method steps for certification configuration corresponding to one or more operator(s) and one or more certification(s).
Note: The figures are not drawn to scale, are provided as illustration only, and serve only for better understanding, not for defining the scope of the invention. No limitations of any features of the invention should be inferred from these figures.

### DETAILED DESCRIPTION

Certain terms will be used in this patent application, the formulation of which should not be interpreted to be limited by the specific term chosen, but as to relate to the general concept behind the specific term.

As used herein, the terms 'comprises,' 'comprising,' 'includes,' 'including,' 'has,' 'having' or any other variation thereof, are intended to cover a non-exclusive inclusion of features.

The terms **'patient sample'** and **'biological sample'** refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semisolid biological material can be rendered liquid by dissolving or suspending it with a suitable liquid medium. In some embodiments, the sample can be suspected to contain a certain antigen or nucleic acid.

The term **'analyzer'** as used herein encompasses any apparatus for obtaining a measurement value from a patient sample. For example, the analyzer can measure light absorption, fluorescence, electrical potential or other physical or chemical characteristics of a reaction or physical process to provide the measurement data. Often such patient samples are treated before analytical testing can be done. Blood sampled from a patient can be e.g. centrifuged to obtain serum or treated with anti-coagulants to obtain plasma.

Analytical testing by an analyzer has the goal to determine the presence and/or concentration of an analyte in a patient sample. The term **'analyte'** is a general term for substances for which information about presence and/or concentration is intended. Examples of analytes are e.g. glucose, coagulation parameters, endogenic proteins (e.g. proteins released from the heart muscle), metabolites, nucleic acids and so on. The term **'patient health parameter'** as used herein encompasses any aspect of a patient's physiology that is measurable or indicated by an analysis of a patient or patient sample for one or more analyte(s), for example blood pressure, blood oxygen level, body temperature, Urine specific gravity, or the like.

The term **'analytical data'** as used herein encompasses any data that is descriptive of a result of a measurement of one or more patient health parameter(s) performed by a point of care analyzer of the biological sample that has been analyzed. In case of a calibration the analytical data can include the calibration result, i.e. calibration data. In particular, the analytical data can include an identifier of the patient sample for which the analysis has been performed and data being descriptive of a result of the analysis, such as measurement data.

The term **'workflow'** as used herein encompasses any task carried out by a human a machine, or a combination of a human and a machine that includes one or more steps, such as for maintenance or operation of a system or one of its system components.

The term **'step of a workflow'** as used herein encompasses any activity belonging to a workflow.

The term **'authentication data'** as used herein encompasses any kind of data suitable to identify an operator/user, such as a user name/user ID and/or password, a security token, a biometric identifier(s) or the like.

The term **'authentication and authorization unit'** as used herein encompasses any hardware-, firmware- and/or software- based module operable to execute program logic for receiving and processing authentication data. Furthermore, the authentication and authorization unit can include any hardware-, firmware- and/or software- based module operable to execute program logic for determining, if the authenticated user/operator possesses the authorization to access a requested feature/ data/ resource / process/ or the like.

The term **'point of care POC'** point of care POC or '**point of care POC environment'** as used herein is defined to mean a location on or near a site of patient care where medical or medically related services such as medical testing and/or treatment are provided, including but not limited to hospitals, emergency departments, intensive care units, primary care setting, medical centers, patient homes, a physician's office, a pharmacy or a site of an emergency.

The term **'point of care testing POCT'** as used herein encompasses analysis of one or more patient(s) or patient sample(s) for one or more patient health parameters in a point of care POC environment. Point of care testing POCT can be often accomplished through the use of transportable, portable, and handheld instruments, but small benchtop analyzers or fixed equipment can also be used when a handheld device is not available- the goal being to collect the patient health parameter and obtain analytical data in a (relatively) short period of time at or (relatively) near the location of the patient.

The term **'point of care analyzer'** as used herein encompasses any analyzer used in a point of care POC environment, such as (but not limited to) blood glucose testing, coagulation testing, blood gas and electrolytes analysis, urinalysis, cardiac markers analysis, hemoglobin diagnostics, infectious disease testing, cholesterol screening or nucleic acid testing. Results may be viewed directly on the point of care analyzer(s) or may be sent to the point of care testing POCT system and displayed in a Laboratory Information System with central lab results, or alongside other results in a Hospital Information System.

The term **'portable computing device'** as used herein encompasses any electronic appliance that can be moved from one location to another without the need of using a tool to sever a connection of the appliance with another appliance, in particular, any handheld battery powered mobile appliance, including but not limited to a cellular telephone, a satellite telephone, a pager, a personal digital assistant ("PDA"), a smartphone, a navigation device, a smartbook or reader, a combination of the aforementioned devices, a tablet computer or a laptop computer.

The term **'communication network'** as used herein encompasses any type of wired or wireless network, including but not limited to a WIFI, GSM, UMTS or other wireless digital network or a wired network, such as Ethernet or the like. For example, the communication network may include a combination of wired and wireless networks.

The term **'server'** as used herein encompasses any physical machine or virtual machine having a physical or virtual processor, capable of accepting requests from and giving responses accordingly. It shall be clear to a person of ordinary skill in the art of computer programming that the term machine may refer to a physical hardware itself, or to a virtual machine such as a JAVA Virtual Machine JVM, or even to separate virtual machines running different Operating Systems on the same physical machine and sharing that machine's computing resources. Servers can run on any computer including dedicated computers, which individually are also often referred to as 'the server' or shared resources such as virtual servers. In many cases, a computer can provide several services and have several servers running. Therefore, the term server shall encompass any computerized device that shares a resource with one or more client processes.

The term **'subserver'** as used herein encompasses any type of hardware-, firmware- and/or software- based module which may be functionally and/or structurally part of a server. Therefore any server which is itself part of a server may be referred to as a subserver. Nevertheless any server as defined above which can be part of a system may be referred to as a subserver.

The term **'hardware management server'** as used herein encompasses any type of server which is configured to manage and to provide connectivity to one or more point of care analyzers. A hardware management server can also be referred to as a point of care POC data management system or generically a middleware. In particular, the term 'hardware management server' as used herein encompasses IT solutions for the point of care POC environment, such as the cobas IT 1000 application from Roche Diagnostics (Mannheim, Germany). According to certain embodiments, the hardware management server can provide not only connectivity for point of care analyzers but also management of test results, operators, quality controls, and analyzers.

The term **'remote configuration server'** as used herein encompasses any type of server configured to provide remotely accessible configuration services, in particular configuration services accessible through a portable computing device.

The term **'server interface'** as used herein encompasses any hardware-, firmware- and/or software- based module operable to execute program logic to allow communication with an external entity (such as a server or another interface). An interface is a boundary across which two separate components of a system exchange data/ information. The exchange can be between software, computer hardware, peripheral devices, humans and combinations of these.

The term **'expose'** as used herein - with reference to a server exposing an interface - refers to a server enabling a separate entity (e.g. another server/ subserver or another interface) access to certain of its features, data, functionality via the interface.

The term **'adapter unit'** as used herein encompasses any type of server/ subserver or any hardware-, firmware- and/or software- based module configured to connect multiple software and/or hardware entities. The functionalities and configuration of the adapter according to the disclosed system / method shall be described in corresponding paragraph(s) below.

The term **'user interface'** as used herein encompasses any suitable piece of software and/or hardware for interactions between an operator and a machine, including but not limited to a graphical user interface for receiving as input a command from an operator and also to provide feedback and convey information thereto. Also, a system /device may expose several user interfaces to serve different kinds of users/ operators.

In the field of bedside testing or point of care testing POCT, the testing is done on patients typically by nurses, medical staff or doctors but also pharmacists who are collectively referred to as **'operator**(s)' herein. However, anyone who possesses the required certification may be an operator. A point of care coordinator POCC may be at the same time an operator of point of care analyzer(s) and also an operator of point of care analyzer(s) may be at the same time a point of care coordinator POCC point of care coordinator POCC and thus user of portable computing device(s).

The term **'certification'** as used herein encompasses any form of confirmation of certain characteristics (such as training and/or examination and/or educational background and/or accreditation) of an operator. In particular a certification as disclosed herein is not restricted to embodiments which are formally titled "certification" or physical embodiments (such as a printed certification) having a related title. According to embodiments of the disclosed system/ method, certification(s) are provided by an entry of an operator on list(s) of certified operators allowed to perform a job/ task / workflow or step of a workflow using one or more of the point of care analyzers. The certification(s) according to embodiments of the disclosed system/ method may be permanent and/or time-restricted certifications, meaning that the certification corresponding to an operator becomes invalid after a certain period of time. After a certification becomes invalid and the respective operator needs to become certified again (by taking a training and/or passing a (re)examination), otherwise that operator may no longer use the respective point of care analyzer(s) or certain features/ functions thereof. The term **'system certification'** as used herein encompasses a certification stored on a server. According to embodiments, the system certification relates to all point of care analyzers of the point of care testing POCT system. According to further embodiments, the system certification relates to one or more point of care analyzers of a certain type, class, namely point of care analyzers having at least one common characteristic. Examples of common characteristics of one or more point of care analyzers are: point of care analyzers being capable of performing the same or similar analyses of patient(s) or patient sample(s); point of care analyzers requiring the same or similar operator training/ examination / certification; point of care analyzers from one manufacturer; point of care analyzers at the same healthcare facility, etc. The term **'analyzer certification'** as used herein encompasses any certification stored on a point of care analyzer. According to some embodiments, the analyzer certification stores any kind of representation of the operators (such as a list of operator identifiers) authorized to perform at least one job/ task / workflow or step of a workflow using that particular point of care analyzer, in particular operators authorized to analyze one or more patient sample(s) using that particular point of care analyzer. According to certain embodiments, each analyzer certification can be specific to one particular point of care analyzer. According to further embodiment(s), each analyzer certification can be specific to one or more point of care analyzers of a certain type, class. According to even further embodiment(s), the analyzer certification(s) may be identical to the system certification(s), but stored locally on the point of care analyzer(s).

Since point of care testing POCT performed near the patient leads directly to diagnostic and therapeutic decisions, a point of care testing POCT system advantageously meets multiple requirements (similar to requirements in laboratory testing, but often within shorter times). Examples of such requirements include:
- Provide accurate and timely analyses and associate them with the correct patient;
- Ensure that operators are competent / certified for the use of the point of care testing POCT system;
- Ensure proper operation, availability and configuration of the analyzers;
- Provide reports that are useful to the clinician treating the patient; and
- Document testing and Quality Control QC for audit purposes.

Point of care testing POCT can be performed using various point of care analyzers such as (but not limited to) analyzers for glucose, coagulation, blood gas, urinalysis, cardiac and molecular testing. Results may be viewed directly on the point of care analyzer(s) or may be sent to the point of care testing POCT system and displayed in a Laboratory Information System with central lab results, or alongside other results in a Hospital Information System.

Point of care analyzers are commonly managed by a server, and in particular, a hardware management server, also referred to as point of care POC data management systems. Such a server provides connectivity for point of care analyzers and management of test results, operators, quality controls, and analyzers.

Management of point of care testing POCT can be challenging - there can be dozens of sites, hundreds of point of care testing POCT devices/kits, and thousands of operators to manage to assure quality of testing. One challenge in developing a strategy to manage point of care testing POCT usually involves building a competent interdisciplinary point of care POC management team including the laboratory, physicians, and nurses. The point of care POC team should usually hold the responsibility for determining the test menu, selecting technologies, establishing policies and procedures, ensuring training and regulatory compliance, and providing advisory assistance to the end operators of point of care POC technologies. After establishing a point of care POC team, a management structure is often built that is responsible to implement new initiatives and to perform corrective action where necessary. The point of care analyzers of a point of care testing POCT system are generally managed by one or more Point of care coordinator(s) POCC. The point of care coordinator POCC is responsible for ensuring that all analyzers are up and running; that all operators are able to use the analyzer(s); know where the analyzers and operators are; and make sure the system is compliant with the regulatory requirements.

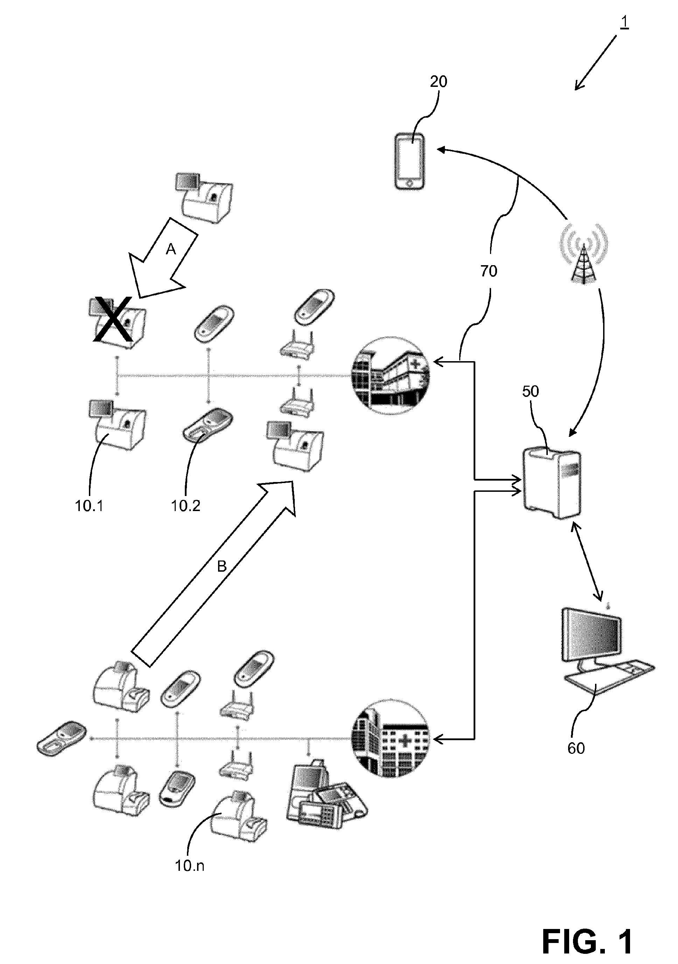

As illustrated on figure 1 for example, the point of care testing POCT system 1 as disclosed herein includes one or more point of care analyzer(s) 10.1 - 10.n, a portable computing device 20 and a server 50 communicatively connected by a communication network 70. In particular, the communication network 70 configured to communicatively connect the one or more point of care analyzer(s) 10.1 - 10.n and the portable computing device 20 with the server 50.

The point of care analyzer(s) 10.1 - 10.n are provided and configured for analyzing one or more patient sample(s) in order to measure one or more patient health parameter(s). According to disclosed embodiments, point of care analyzer(s) 10.1 - 10.n include transportable, portable, and handheld instruments, but small bench analyzers or fixed equipment as well, such as (but not limited to) blood glucose testing, coagulation testing, blood gas and electrolytes analysis, urinalysis, cardiac markers analysis, hemoglobin diagnostics, infectious disease testing, cholesterol screening or nucleic acid testing NAT. Several functional and/or operational aspects of the point of care analyzer(s) 10.1 - 10.n are configurable/ customizable using one or more analyzer parameter(s).

In order to identify a particular point of care analyzer(s) 10.1 - 10.n, each is provided with an analyzer identifier, in particular in form of an identifier tag, such as a barcode and/ or an RFID tag but can be a serial number as well.

The server 50 is provided and configured for storing system parameter(s) corresponding to the one or more point of care analyzer(s) 10.1 - 10.n. Some of these system parameters are common for more than one point of care analyzers while some system parameters are specific to one single individual point of care analyzer 10.1 - 10.n.

The at least one system parameter and/or analyzer parameter include (but are not limited to) one or more of the following:
- Analyzer specific parameters:
   - Formatting setting(s)
   - Language setting(s),
   - Date/Time format setting(s)
   - Shutdown/ Sleep/ Hibernate / Logout timeout
   - Connection configuration (e.g. wlan authentication data)
   - Analyzer Status: in use, backup, etc.
- Security parameters
   - Authentication mechanism
   - Login mechanism (only user ID, user ID and Pwd or Barcode scanning)
- Patient identification/ mapping parameters
   - Patient identification mechanism
   - Patient ID mapping
- Measurement parameters
   - Default measurement unit(s)
   - Workflow definitions) (e.g. force having comments on results or set them optional)
   - Ranges (such as reference ranges)
- Quality Control QC parameters
   - QC Lockout
   - Lot verification
   - QC result display
- Location specific parameters
   - Physical location: Healthcare Facility, Building, Floor, Unit, Room
   - Logical location: Emergency room, intensive care units, primary care setting, medical center, patient home, a physician's office, a pharmacy or a site of an emergency
   - Location-specific authentication and authorization data.

In certain embodiments of the disclosed system/ method, the server 50 is configured to:
- retrieve analytical data from the one or more point of care analyzer(s) 10.1 - 10.n such as data representing the measurement of patient health parameter(s);
- update program data of the one or more point of care analyzer(s) 10.1 - 10.n such as a software update.

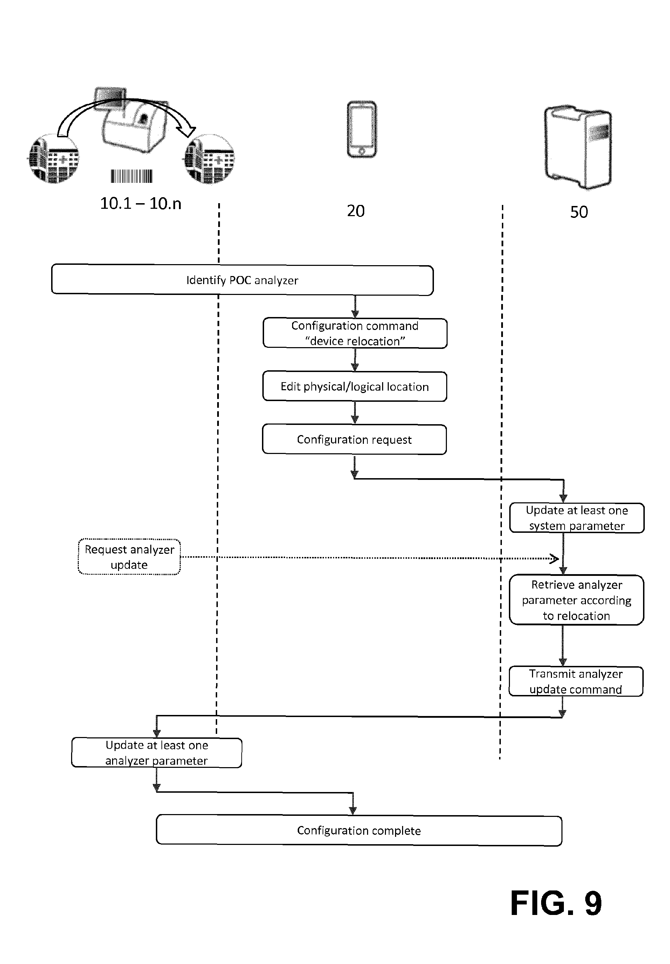

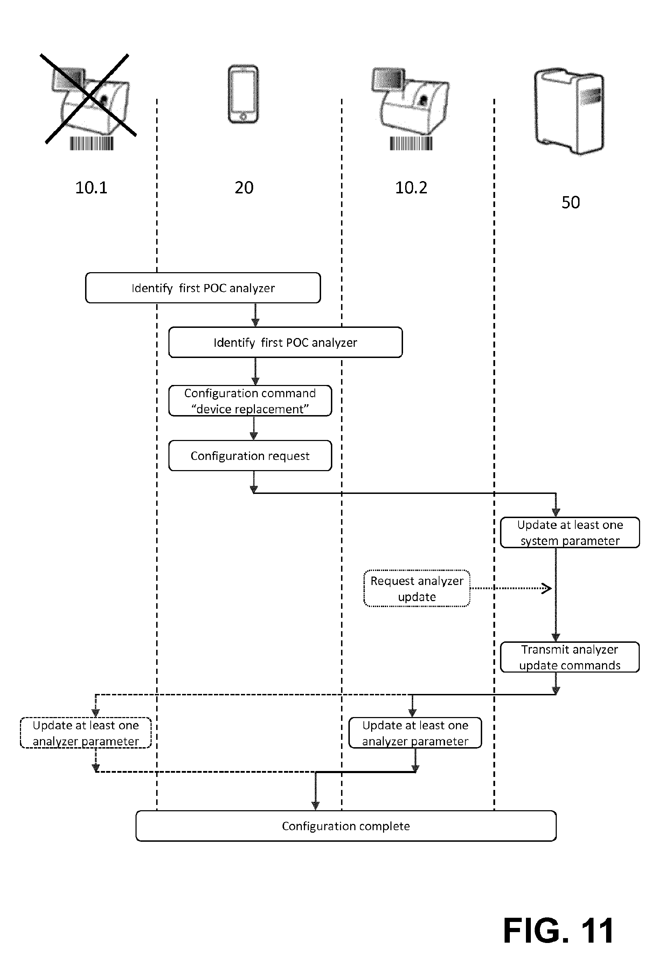

The block arrows A and B of Figure 1 illustrate an analyzer replacement respectively an analyzer relocation workflow as described with reference to the use case diagrams of figures 9 and 11.

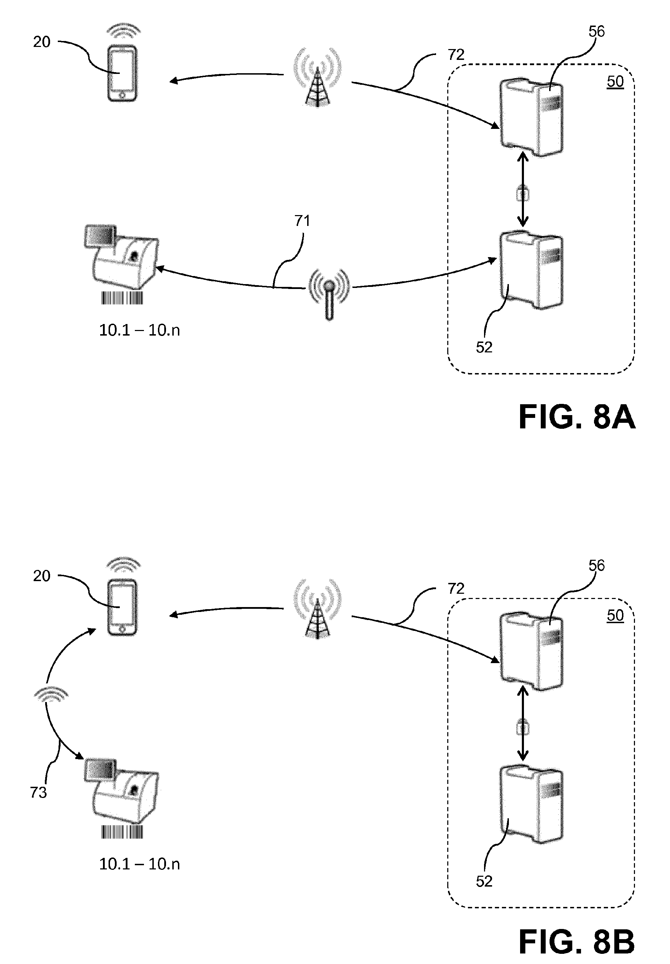

As illustratively shown on Figure 1, the communication network 70 is laid out / configured to communicatively connect the one or more point of care analyzer(s) 10.1 - 10.n and the portable computing device 20 with the server 50, Figures 8A to 8D illustrate different embodiments thereof.

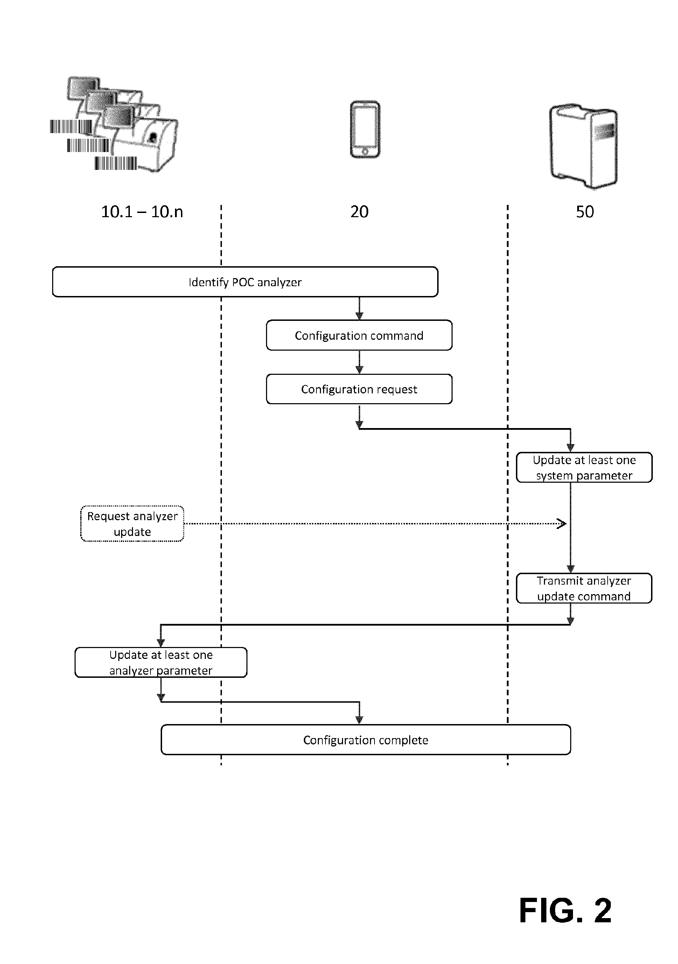

Figure 2 shows a use case diagram of an embodiment of the disclosed method for configuration of a point of care testing POCT system 1. The symbols on the top of the diagram illustrate the actors of the workflow - the point of care analyzers 10.1 - 10.n, the portable computing device 20 and the server 50, while the steps of the workflow are shown below, illustrating the "involvement" of the actors in the respective workflow step(s). Workflow steps of particular embodiment(s) only are illustrated with dotted lines.

In a step, the portable computing device 20 identifies the point of care analyzer(s) 10.1 - 10.n on basis of the respective analyzer identifier. Corresponding to the particular analyzer identifier, the portable computing device 20 can include an identifier reader such as a barcode reader and/or an RFID reader to read the identifier tag and/or a user interface for inputting a serial number of the one or more point of care analyzer(s) 10.1 - 10.n. Alternatively an imaging device (such as a camera) of the portable computing device 20 is configured to identify the point of care analyzer(s) 10.1 - 10.n. It shall be noted that the portable computing device 20 can be configured such that it can identify more than one point of care analyzer(s) 10.1 - 10.n at the same time or successively and thus initiate a reconfiguration of more than one point of care analyzers 10.1 - 10.n at the same time. The more than one point of care analyzers 10.1 - 10.n identified are referred to as the identified point of care analyzer(s) 10.1 - 10.n.

In order to initiate/ start a configuration workflow (process), a configuration command is given (by an operator). Therefor the portable computing device 20 is provided with a user interface 22 configured to receive the configuration command. According to embodiments of the user interface 22, the configuration command may be a push of a button (physical or screen button) a voice command, a selection in a menu, etc. The configuration command may be any form of input from an operator to initiate a configuration workflow (process).

Triggered by the configuration command, the portable computing device 20 generates a configuration request including the analyzer identifier of the identified point of care analyzer(s) 10.1 - 10.n. After generating it (optionally after confirmation from the operator), the portable computing device 20 transmits the configuration request to the server 50. Therefore the configuration request can be described as a translation of the configuration command from the operator into a request signal to the server 50.

The server 50 is configured to receive the configuration request, the transmission thereof being performed using the communication network 70. After receiving it, the server 50:
- updates at least one system parameter corresponding to the identified point of care analyzer(s) 10.1 - 10.n; and
- transmits an analyzer update command including at least one analyzer parameter update to the identified point of care analyzer(s) 10.1 - 10.n.

The above steps by the server 50 shall now be described in greater detail. On one hand, the server 50 is configured to update at least one system parameter corresponding to the identified point of care analyzer(s) 10.1 - 10.n. The server 50 retrieves (looks up) the corresponding system parameter(s) as the configuration request generated by the portable computing device 20 can include the analyzer identifier(s). Depending on the configuration command / configuration request (as it shall be described in following paragraphs of this specification), the server 50 updates the appropriate system parameter(s).

In order to ensure that the configuration command (as a complete workflow) is implemented not only on the server 50 but across the point of care testing POCT system 1, the server 50 is configured to transmit an analyzer update command(s) including at least one analyzer parameter update to the identified point of care analyzer(s) 10.1 - 10.n which in turn are configured to receive the analyzer update command and to update at least one analyzer parameter according to the corresponding analyzer parameter update. According to embodiment(s) of the disclosed system/ method and according to the particular point of care analyzer(s) 10.1 - 10.n, the updating of an analyzer parameter may become effective immediately, upon the next start-up/ restart/ docking etc. of the analyzer.

In other words, the configuration command given via the user interface 22 of the portable computing device 20 initiates a system-wide configuration workflow, wherein within said configuration workflow, the portable computing device 20, the server 50 and the point of care analyzers 10.1 - 10.n collaborate (via the communication network) to ensure that required parameters (system parameters and analyzer parameters alike) are updated across the point of care testing POCT system 1. This approach has the advantage that a single configuration command can be given from a remote location (remote meaning a location different from the server) in order to (re)configure the point of care testing POCT system 1 - including any number of point of care analyzers, the point of care testing POCT system 1 being configured such as to implement the configuration command without the need for additional actions /steps by the operator. Thus both operator effort and probability of operator error are greatly reduced, as there is no need for the operator to individually update system parameters on the server 50, identify the corresponding analyzer parameters and then update the respective point of care analyzers 10.1 - 10.n. These steps are taken over by the disclosed system / method. Therefore embodiments of the disclosed system / method are particularly advantageous over existing solutions, which commonly perform only the update of the system parameters on a server, wherein the operator is responsible to perform the update of the analyzer parameters of the respective point of care analyzer(s).

According to embodiments of the disclosed system/ method, the steps of:
- the server 50 transmitting an analyzer update command;
- the point of care analyzer(s) 10.1 - 10.n receiving the analyzer update command; and
- the point of care analyzer(s) 10.1 - 10.n updating of at least one analyzer parameter
   are initiated by one or more point of care analyzer(s) 10.1 - 10.n requesting an analyzer update. Correspondingly, the one or more point of care analyzer(s) 10.1 - 10.n are configured to request an analyzer update on occurrence(s) of certain event(s) and/or at regular intervals. According to embodiments of the disclosed system/ method, such event(s) include (but are not limited to) a login by an operator; a startup/ shutdown/ docking/ undocking (into a docking station) of the point of care analyzer 10.1 - 10.n. According to embodiments of the disclosed system/ method, the regular intervals (when the one or more point of care analyzer(s) 10.1 - 10.n are configured to request an analyzer update) are chosen to coincide with work shift changes of the operators or with time intervals when - based on historical data - the point of care analyzers are least expected to be used. Thus the regular intervals are configured such as to minimize unavailability (or predicted unavailability) of the point of care analyzer(s) 10.1 - 10.n.

To summarize the analyzer update(s) - according to embodiment(s) of the disclosed system/ method - the analyzer update(s) may be implemented using "server push" technology (that is server initiated) and/or a "client pull" technology (client - in this case point of care analyzer initiated). The server push implementation of the analyzer update is advantageous for point of care analyzers 10.1 - 10.n which are continuously communicating with the server 50, while the client pull implementation of the analyzer update is advantageous for point of care analyzers 10.1 - 10.n which communicate with the server 50 only on an event basis, such as periodically and/or upon a login by an operator and/or upon startup and/or upon shutdown of the respective point of care analyzer 10.1 - 10.n.

Embodiments of the disclosed system / method are particularly advantageous as they allow a decentralized control of point of care analyzers by a portable computing device. The disclosed solution allows communication with hardware management software (such as the COBAS IT 1000 of Roche Diagnostics) through a portable computing device, making it possible to (re)configure point of care analyzer(s) and at the same time also initiate the corresponding server update(s). This combined update of both system parameter(s) and analyzer parameter(s) ensures consistent (re)configuration of the point of care testing POCT system within a single workflow. The exposure by the point of care testing POCT system of such workflows for the (re)configuration of point of care analyzers remotely by a portable computing device are collectively referred to as **'remote configuration services'.**

Further embodiments of the disclosed point of care testing POCT system are configured to enable efficient workflow solution(s) for the configuration management of point of care analyzer(s), which system architecture is optimized such that existing hardware managements server(s) / systems need little to no modifications. These further embodiments address the difficulties in the implementation of configuration management workflows for point of care analyzer(s) due to the high degree of complexity and lack of modularity and adaptability of existing system architectures for point of care testing POCT systems. In particular, embodiments herein disclosed are configured to enable remote configuration services of point of care analyzers from different types, vendors which are connected to different standard and/or proprietary hardware management systems, by handling authentication and authorization data with respect to configuration management across different platforms.

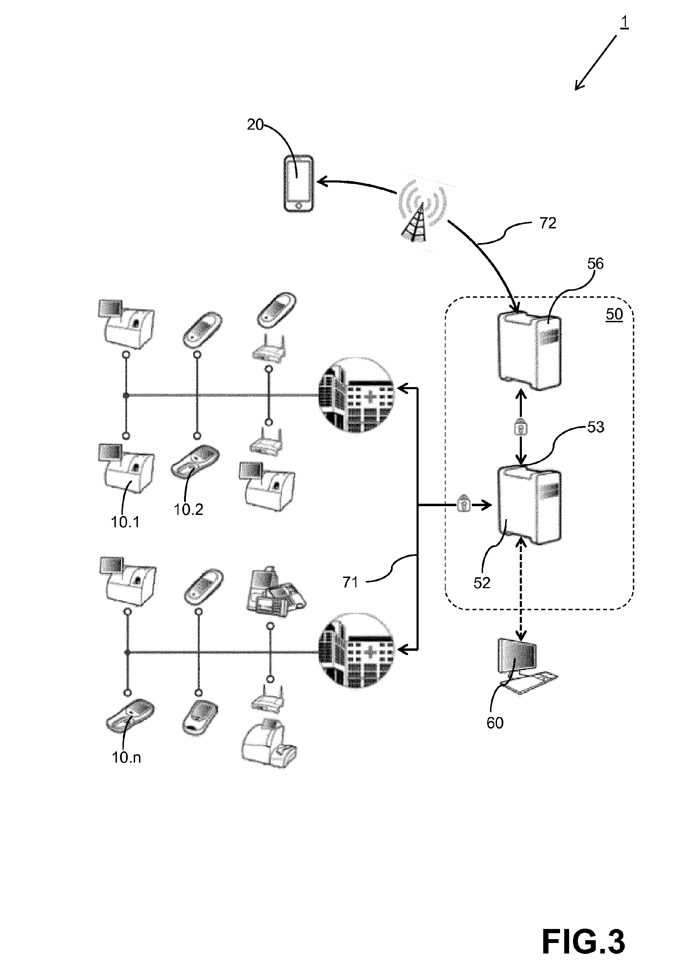

As illustrated on figure 3, according to further embodiments of the disclosed point of care testing POCT system the functionalities of the server 50 are distributed between a hardware management server 52 and the remote configuration server 56. It shall be noted that according to various embodiments, the hardware management server 52 and a remote configuration server 56 may or may not be one and the same hardware-, firmware- and/or software unit.

The hardware management server 52 is provided and configured for storing the system parameter(s) (see definition above) corresponding to the point of care analyzer(s) 10.1 - 10.n. Also, it is the hardware management server 52 that is communicatively connected to the point of care analyzer(s) 10.1 - 10.n, via the communication network 70.

As shown on figure 3, a management console 60 may be provided to access the hardware management server 52.

According to embodiments of the disclosed system/ method, the hardware management server 52 can be configured to expose a hardware management server interface 53 for communication with the remote configuration server 56.

The disclosed embodiments of the remote configuration server 56 allow consumer systems - that is the hardware management server(s) 52 to resolve what are the resources and actions exposed. This supports easy development of interfaces which have to connect to the remote configuration server 56.

While not shown on the figures, the hardware management server 52 can be further configured to perform one or more of the following: retrieve analytical data from the point of care analyzer(s) 10.1 - 10.n; update program data of the point of care analyzer(s) 10.1 - 10.n; transfer of authentication data update(s) and/or certification update data of the point of care analyzer(s) 10.1 - 10.n.

According to embodiments of the disclosed system/ method, the communication network 70 can include:
- a point of care POC communication network area 71 for communicatively connecting the point of care analyzer(s) 10.1 - 10.n with the hardware management server 52;
- a remote configuration network area 72 for communicatively connecting the portable computing device 20 with the remote configuration server 56.

Particulars of the communication network 70 are described with reference to figures 8A through 8D.

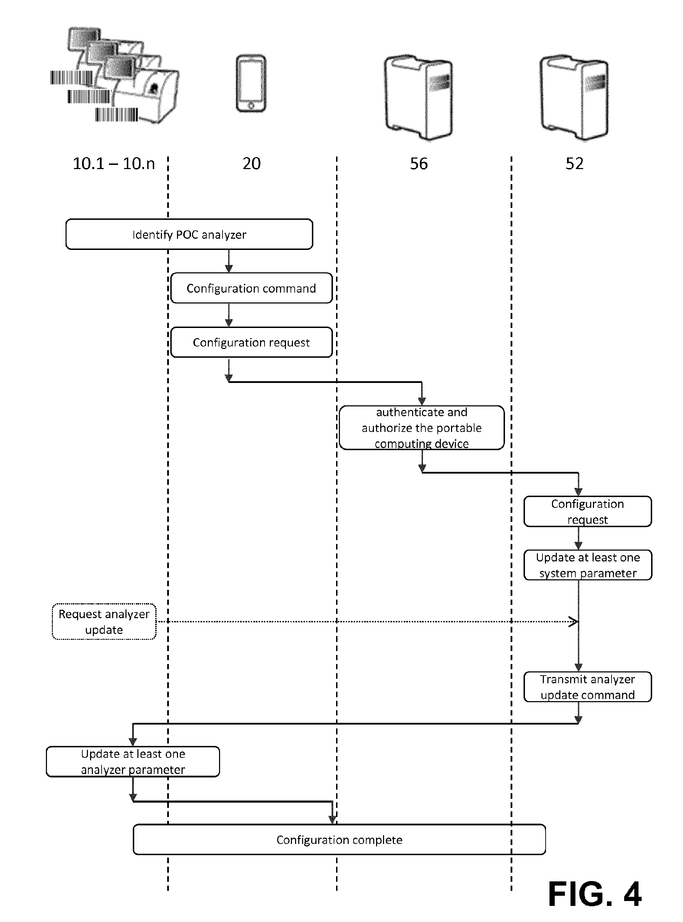

Figure 4 shows a use case diagram of an embodiment of a disclosed method for configuration of a point of care testing POCT system 1 including a remote configuration server 56 and a hardware management server 52.

In a step, the portable computing device 20 identifies the point of care analyzer(s) 10.1 - 10.n on basis of the respective analyzer identifier. Thereafter, in order to initiate/ start a configuration workflow (process), a configuration command is given (by an operator) via a user interface 22 of the portable computing device 20.

Triggered by the configuration command, the portable computing device 20 generates a configuration request including the analyzer identifier of the identified point of care analyzer(s) 10.1 - 10.n. After generating it (optionally after confirmation from the operator), the portable computing device 20 transmits the configuration request to the remote configuration server 56.

Being communicatively connected by the communication network 70 to the portable computing device 20, the remote configuration server 56 is provided to authenticate and authorize the portable computing device 20. The authentication of the portable computing device 20 is based on authentication data, such as a user name/user ID and/or password, a security token, a biometric identifier(s) or the like to authenticate the portable computing device 20 and/or an operator thereof. After authentication, the remote configuration server 56 determines if the authenticated portable computing device 20, respectively a user thereof, possesses the authorization to access a requested feature/ data/ resource / process/ or the like, in particular whether the authenticated portable computing device 20 is allowed to carry out the workflow initiated by the configuration request.

Since the task of authenticating and authorizing the portable computing device 20 is handled by the remote configuration server 56, the management of the remote configuration services (via the portable computing device 20) is handled by the remote configuration server 56 and may be transparent to the hardware management server 52 and the point of care analyzer(s) 10.1 - 10.n. Such embodiments of the disclosed system/ method are advantageous as the use of a dedicated remote configuration server 56 for connecting the portable computing device(s) 20 allows implementing remote configuration of existing point of care testing POCT systems. Thus, in particular embodiments, remote configuration of existing point of care testing POCT systems may be implemented fully agnostic to the hardware management server 52 and the point of care analyzer(s) 10.1 - 10.n. In other words, there is no need to change the hardware management server 52 and the point of care analyzer(s) 10.1 - 10.n. On the other hand, the underlying hardware management server 52 can be exchanged, substituted or scaled without affecting the remote configuration server 56 and thus the remote configuration of the point of care testing POCT system 1 via portable computing device(s) 20.

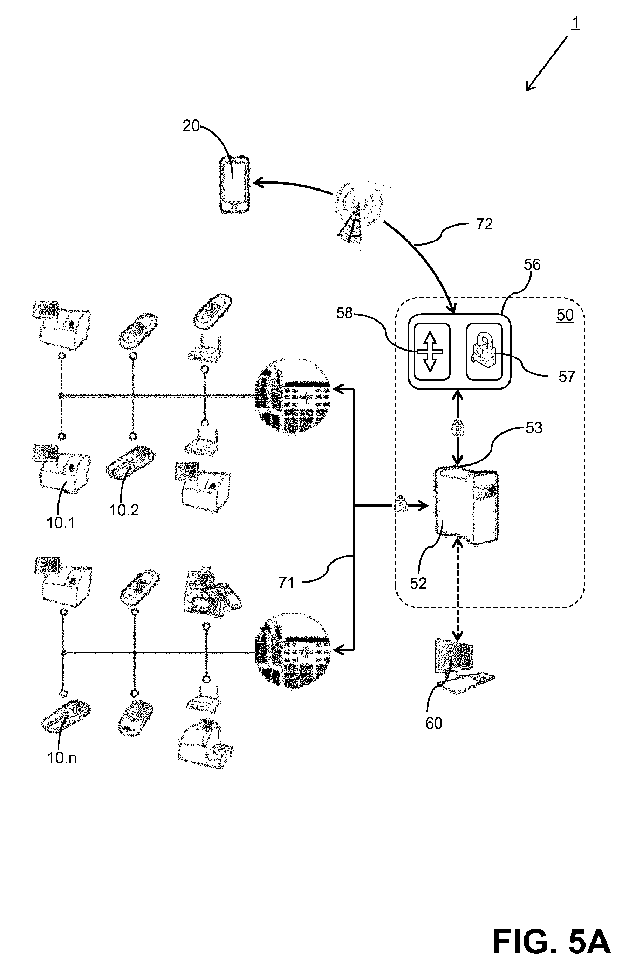

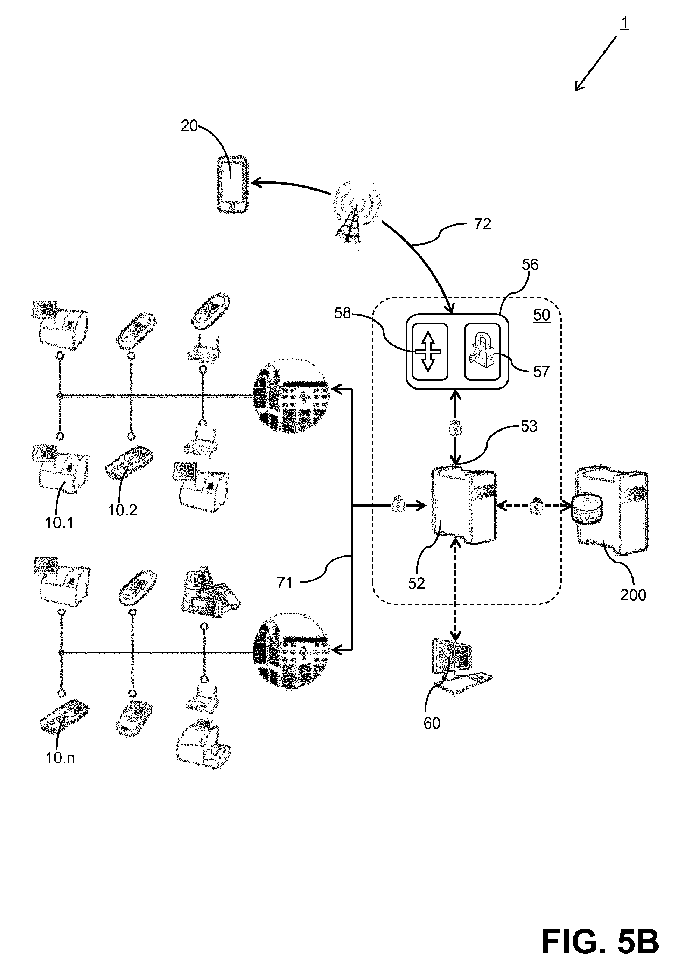

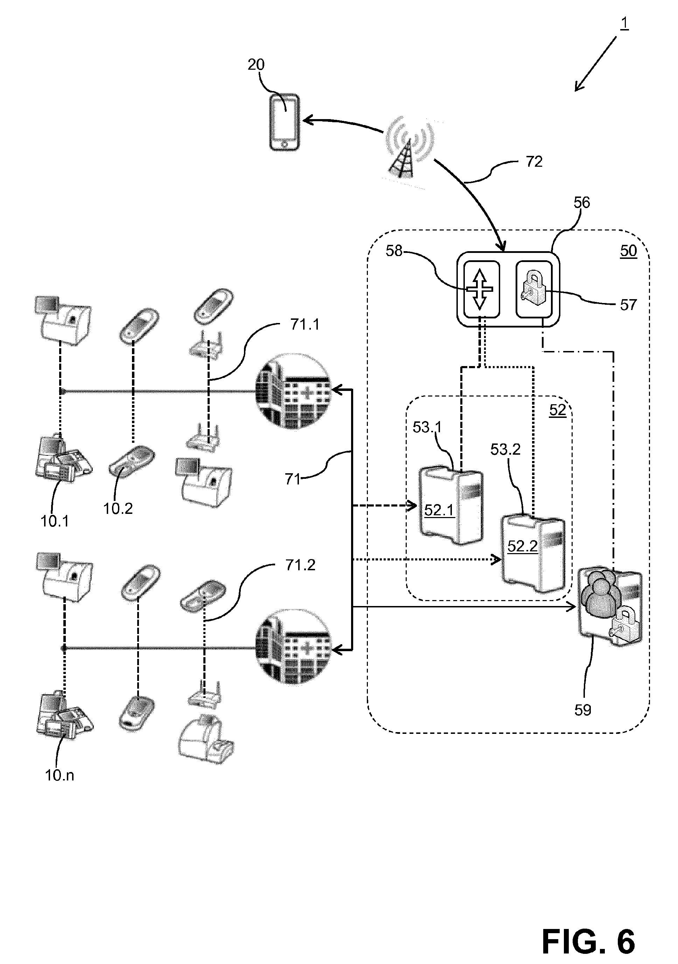

After successful authentication and authorization of the portable computing device 20, the remote configuration server 56 receives/ accepts the configuration request from the portable computing device 20. Thereafter - being communicatively connected by the communication network 70 to the hardware management server 52 - the remote configuration server 56 is further configured to forward the configuration request(s) to the hardware management server 52. As it shall be described in relation to figures 5A, 5B and 6, according to particular embodiments, the remote configuration server 56 is configured to adapt the configuration request received from the portable computing device 20 before transmitting the configuration request to the hardware management server 52, in accordance with the particular hardware management server 52.

Therefore, according to embodiments of the disclosed system/method, the remote configuration server 56 takes over both the communication with and also the authentication/ authorization of the portable computing device(s) 20. Thus it is the remote configuration server 56 which exposes the remote configuration services to the portable computing device(s) 20, ensuring itself compatibility with the particular hardware management server 52.

The hardware management server 52 then receives the configuration request from the remote configuration server 56, the transmission thereof being performed using the communication network 70. After receiving it, the hardware management server 52 updates at least one system parameter corresponding to the identified point of care analyzer(s) 10.1 - 10.n. In order to ensure that the configuration command (as a complete workflow) is implemented not only on the hardware management server 52 but across the point of care testing POCT system 1, the hardware management server 52 is configured to transmit an analyzer update command(s) including at least one analyzer parameter update to the identified point of care analyzer(s) 10.1 - 10.n which in turn are configured to receive the analyzer update command and to update at least one analyzer parameter according to the corresponding analyzer parameter update. According to embodiment(s) of the disclosed system/ method and according to the particular point of care analyzer(s) 10.1 - 10.n, the updating of an analyzer parameter may become effective immediately, upon the next start-up/ restart/ docking etc. of the analyzer.

It shall be noted, that particulars described with reference to the embodiments of the system as shown on figure 1 respectively the method as shown on figure 2 apply mutatis mutandis to the embodiments of the system as shown on figures 3 and 5A through 6, respectively the method as shown on the figures 4, 9, 11 and 13, namely embodiments including a dedicated hardware management server 52 and a dedicated remote configuration server 56.

Figure 5A shows an illustrative diagram of a further embodiment of the disclosed point of care testing POCT system 1, the remote configuration server 56 further including an authentication and authorization unit 58 and an adapter unit 57.

The authentication and authorization unit 57 is provided and configured to authenticate and authorize the portable computing device 20 and/or a point of care coordinator POCC via the portable computing device 20 (see above a detailed description of the authentication and authorization) with the remote configuration server 56.

The adapter unit 58 is provided for and configured to adapt the configuration request (received from the portable computing device 20) to the hardware management server interface 53 before transmitting the configuration request to the hardware management server 52. Adapting a configuration request(s) to an interface refers to enabling communicative data exchange with the particular interface.

Therefore, the term **'adapt'** or **'adapting'** as used herein with reference to the adapter unit 58 adapting a configuration request(s) to an interface can include (but is not limited to) one or more of the following:
- reformatting/ encapsulating the configuration request to a format compatible with the target interface, for example to use standards such as Health Level Seven HL7 or Fast Healthcare Interoperability Resources FHIR;
- redirecting the configuration request to the respective hardware management server (see figure 6 and related paragraphs);
- appending authentication and/or authorization data related to the remote configuration server 56 to the configuration request, said authentication and/or authorization data authenticating and authorizing the remote configuration server 56. In other words, the remote configuration server 56 authenticates the portable computing device 20 based on credentials thereof, but forwards the configuration request with its own credentials. This way the remote configuration (via portable computing device(s)) is transparent to the hardware management server 52;
- encrypting the configuration request before transmission;
- compressing the configuration request before transmission, such as compressing the request using standard HTTP technology so that network latency is reduced and performance is improved between the adapter unit 58 and the hardware management server 52.

The modular design shown on figures 5A through 6 allows the system 1 to define multiple adapters based on domain & standard-driven resources so that multiple adapters can be built out of the modules connected to them to fit on different purposes or for being tailored down for simplification purposes. Additionally, the possibility of defining multiple adapters by configuring the modules allows higher scalability using multiple machines for exposing the final interface combining software and platform as service paradigms.

Further units (not shown on the figures) of the remote configuration server 56 can include one or more of the following:
- Common Management
- Device Management
- Practitioner Management
- Organization Management

Figure 5B shows an embodiment of the disclosed point of care testing POCT system 1 as communicatively connected to a Laboratory Information System LIS and/or Hospital Information system HIS 200. It is readily apparent that the remote configuration services - made available by the disclosed solution including a remote configuration server 56 communicating with the hardware management server 52 - may be implemented without affecting an existing installation of a point of care testing POCT system (with one or more point of care analyzers) and a Laboratory Information System LIS and/or Hospital Information system HIS connected thereto. According to certain embodiments, the existence of the remote configuration server 56 - and thus the remote configuration services via a portable computing device 20 - can be also agnostic to the Laboratory Information System LIS and/or Hospital Information system HIS.

As shown on Figure 6 according to further embodiment(s) of the disclosed point of care testing POCT system 1, the hardware management server 52 includes a first hardware management subserver 52.1 communicatively connected to a first group I of one or more point of care analyzer(s) 10.1 - 10.n and a second hardware management subserver 52.2 communicatively connected to a second group II of one or more point of care analyzer(s) 10.1 - 10.n. Multiple hardware management subservers 52.1, 52.2 are advantageous in point of care POC environments with point of care analyzers of different standards, from different vendors which need to be connected to different standard and/or proprietary hardware management systems. Thus, the first group I of one or more point of care analyzer(s) 10.1 - 10.n may comprise point of care analyzers of one standard while the second group II of one or more point of care analyzer(s) 10.1 - 10.n may comprise point of care analyzers of a further standard.

Correspondingly, the first hardware management subserver 52.1 is configured to transmit the analyzer update command(s) to the one or more point of care analyzer(s) 10.1 - 10.n of the first group; and the second hardware management subserver 52.2 is configured to transmit the analyzer update command(s) to the one or more point of care analyzer(s) 10.1 - 10.n of the second group.

According to embodiments of the disclosed system/ method, the first hardware management subserver(s) 52.1 can be configured to expose a first subserver interface 53.1 for communication with the remote configuration server 56 and the second hardware management subserver(s) 52.2 can be configured to expose a second subserver interface 53.2 for communication with the remote configuration server 56, the first and second subserver interfaces 53.1, 53.2 not being necessarily identical. Correspondingly, the adapter unit 58 of the remote configuration server 56 is configured to adapt the configuration request corresponding to one or more point of care analyzer(s) 10.1 - 10.n of the first group to the first subserver interface 53.1, in order to accommodate the first subserver interface 53.1 and the adapter unit 58 of the remote configuration server 56 is configured to adapt the configuration request corresponding to one or more point of care analyzer(s) 10.1 - 10.n of the second group to the second subserver interface 53.2, in order to accommodate the second subserver interface 53.2. According to further embodiments, the adapter unit 58 is configured to adapt configuration request(s) to particular hardware management subserver(s) respectively their interface(s) using one or more module(s).

Such embodiments are particularly advantageous as they allow the combination of multiple solutions (hardware management servers) by exposing or aggregating their interfaces based on modules of the adapter unit 58. All the modules are based on plugins so that one can extend the functionality merely by creating new modules.

According to certain embodiments, the first group I of one or more point of care analyzer(s) 10.1 - 10.n connected to a first hardware management subserver 52.1 respectively the second group II of one or more point of care analyzer(s) 10.1 - 10.n connected to second hardware management subserver 52.2 are pre-existing installations of various point of care analyzers, while the remote configuration server 56 is installed as an add-on to provide remote configuration services across all the different groups of point of care analyzers and across the different hardware management subservers. Also, a third (and further) group(s) of one or more point of care analyzer(s) 10.1 - 10.n connected to a third (and further) hardware management subserver 52.x may be added to the system without affecting the other group(s) of point of care analyzer(s), the only requirement being that the adapter unit 58 is further configured to adapt configuration request(s) to the interface of the newly added hardware management subserver.

Therefore, the disclosed system architecture allows both a horizontal and vertical scaling so that point of care testing POCT system(s) with large amounts of consumers can maintain optimal performance and reduce or even eliminate downtime upon scaling/ extension of the system.

According to certain embodiments, as shown on figure 6, in order to connect the different groups of more point of care analyzer(s) 10.1 - 10.n to the respective hardware management subservers 52.1, 52.2, the point of care POC communication network area 71 may comprise:
- a first point of care POC communication subnetwork 71.1 (shown with dashed lines) to communicatively connect the first group of one or more point of care analyzer(s) 10.1 - 10.n and the first hardware management subserver(s) 52.1; and
- a second point of care POC communication subnetwork 71.2 (shown with dotted lines) to communicatively connect the second group of one or more point of care analyzer(s) 10.1 - 10.n and the second hardware management subserver(s) 52.2.

According to various embodiments, the first point of care POC communication subnetwork 71.1 and the second point of care POC communication subnetwork 71.2 may use the same communication channels. In other words, the different subnetworks 71.1, 71.2 may share the same HW infrastructure, the separation into different subnetworks 71.1, 71.2 being implemented on a software level.

Also shown on figure 6, according to certain embodiments of the disclosed system/ method, a directory server 59 can be provided to store authentication and authorization data and configured to allow sharing of the authentication and authorization data between the directory server 59 and one or more of the hardware management server 52, the first hardware management subserver 52.1 and the second hardware management subserver 52.2 for authenticating and authorizing the one or more point of care analyzer(s) 10.1 - 10.n and operator(s) of the one or more point of care analyzer(s) 10.1 - 10.n.

Also shown on figure 6 with dotted-dashed lines, according to certain embodiments of the disclosed system/ method, the authentication and authorization unit 57 of the remote configuration server 56 and the directory server 59 are configured and communicatively connected in order to allow exchange of authentication and authorization data. Thus, the authentication and authorization unit 57 in conjunction with the directory server 59 allow the connection of multiple different services or software solutions so it can be used for providing single sign-on improving efficiency and user experience of the operator(s).

In the following, different embodiments of the communication network 70 shall be described in greater detail. Figure 8A shows a first embodiment of the communication network 70, wherein the portable computing device 20 can be communicatively connected with the remote configuration server 56 by a remote configuration network area 72, while the one or more point of care analyzer(s) 10.1 - 10.n are communicatively connected with the hardware management server 52 using a point of care POC communication network area 71. According to an example illustrated, in particular embodiments, the remote configuration network area 72 can be a mobile telecommunication network (such as a wireless mobile Internet service, for example a 3G, 4G or LTE standard) provided by a mobile data carrier service. On the other hand the point of care POC communication network area 71 (of particular embodiments of the communication network 70) can be a separate network, for example a combination of wired and wireless networks, wherein the healthcare facilities (such as different locations/ buildings /floors) are linked by a wired communication network while the individual point of care analyzer(s) 10.1 - 10.n connect via wireless access points in-between.

As shown on figure 8A, according to certain embodiments disclosed, there is no need for a communication between the portable computing device 20 and the point of care analyzer(s) 10.1 - 10.n as the (re)configuration thereof can be carried out via the hardware management server 52.

Nevertheless, according to a further embodiment of the communication network 70 - as illustrated on Figure 8B, the portable computing device 20 and the point of care analyzer(s) 10.1 - 10.n are communicatively connected by a portable device to analyzer network area 73. In this case, the portable computing device 20 acts as a router/ access point for the point of care analyzer(s) 10.1 - 10.n, "sharing" its network connection therewith. This embodiment is advantageous in use scenarios when the point of care analyzer(s) 10.1 - 10.n do not have direct communication with the server 50. This can be the case for example in remote areas where the point of care analyzers 10.1 - 10.n cannot be connected to a point of care POC communication network area 71 (or not all of them), but the portable computing device 20 does have a network connection via the remote configuration network area 72. In other cases, it might not be economic to provide each point of care analyzer with a network connection. A further scenario - where the portable computing device 20 does have a network connection via the remote configuration network area 72 but not each point of care analyzer - is emergency response, in which case the point of care analyzers 10.1 - 10.n may need to be deployed "in the field" in a short period of time. The disclosed system/ method are advantageous in such scenarios as well, since even without each point of care analyzer 10.1 - 10.n having a network connection of their own, they can all be configured easily and consequently from a portable computing device 20, only this portable computing device 20 needing a network connection.

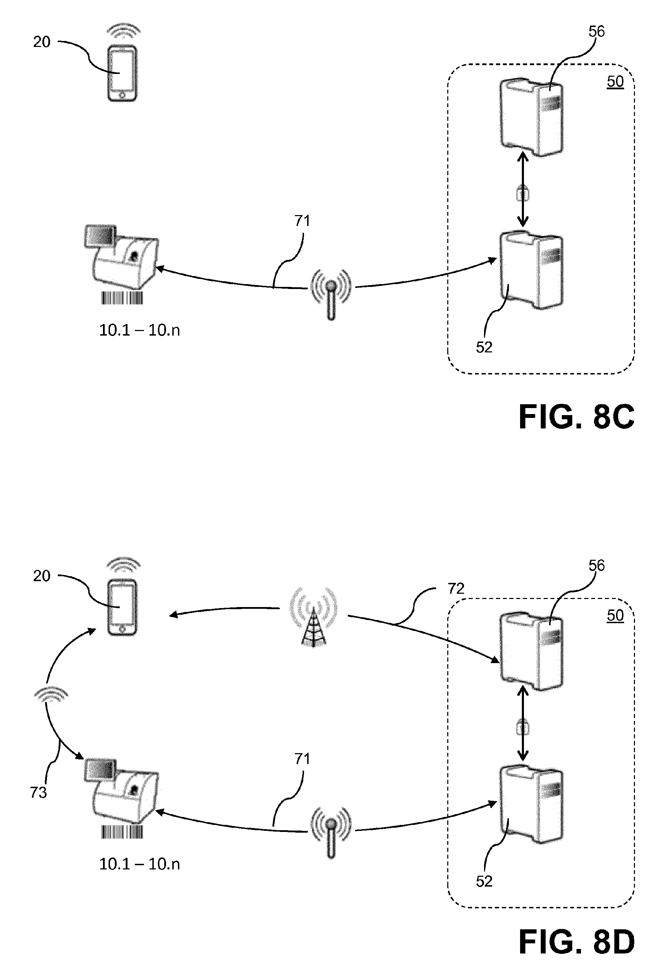

Figure 8C shows a further embodiment of the communication network 70; where a portable device to analyzer network area 73 can be provided to communicatively connect the portable computing device 20 with a point of care analyzer 10.1 - 10.n. In this embodiment the portable computing device 20 is not directly connected with the remote configuration server 56, but via the portable device to analyzer network area 73, the point of care analyzer(s) 10.1 - 10.n acting as a router/ access point for the portable computing device 20, "sharing" their network connection therewith. This embodiment is advantageous in point of care POC environments where the point of care analyzers 10.1 - 10.n are already communicatively connected with the respective hardware management server(s) 52 but there is no mobile telecommunication network (no signal) for the portable computing device 20 to connect to the remote configuration server 56. For example there are point of care POC environments where a mobile telecommunication network is not desired/ permitted, the point of care analyzer(s) 10.1 - 10.n being connected to the respective hardware management server(s) 52 with a wired point of care POC communication network area 71. Nevertheless, a direct communication between the portable computing device 20 and the point of care analyzer(s) 10.1 - 10.n (for example via Bluetooth or IR) would still be possible/ allowable.

Figure 8D shows a further embodiment of the communication network 70, where in addition to a remote configuration network area 72 and a point of care POC communication network area 71, a portable device to analyzer network area 73 can be also provided. This embodiment provides both flexibility and redundancy for the communicative connection of the one or more point of care analyzer(s) 10.1 - 10.n and the portable computing device 20 with the server 50.

It must be pointed out however, that the particular implementation of the communication network 70 (as illustrated on Figures 8A to 8D) can be to a certain degree transparent to the point of care testing POCT system 1 and does not negatively affect the disclosed configuration method(s) as all embodiments of the communication network 70 disclosed herein are configured to communicatively connect the one or more point of care analyzer(s) 10.1 - 10.n with the respective hardware management server(s) 52 and the portable computing device 20 with the remote configuration server 56, be it directly or via different network area(s), such as the point of care POC communication network area 71 and/or the remote configuration network area 72 and/or the portable device to analyzer network area 73.

In the following, particular workflows of configuring a point of care testing POCT system shall be described, the implementation of these workflows being simplified and optimized by the hereby described system architecture of the point of care testing POCT system 1 including a dedicated remote configuration server 56 and a dedicated hardware management server 52 (subservers 52.1-52.x). It shall be recognized, that - according to various embodiments of the disclosed system/ method - such workflows may be added, edited or removed by altering only the remote configuration server 56 but without affecting the hardware management server 52 (or subservers 52.1-52.x). While describing the particular workflows of configuring a point of care testing POCT system, the hardware management server 52 (or subservers 52.1-52.x) and the remote configuration server 56 are collectively referred to as the server 50.

A common task of a point of care coordinator POCC is the relocation of a point of care analyzer(s), necessary due to changed organizational structure or workload fluctuations within the healthcare facilities. The physical relocation of a point of care analyzer(s) usually also triggers the need to update corresponding settings of the server (hardware management server) and also reconfiguration of one or more parameters of the point of care analyzer(s). For example when a point of care analyzer(s) is relocated from a children's hospital ward to an adult's ward, certain parameters of the point of care analyzer(s) may need to be updated accordingly to ensure the analyzer provides accurate results. Furthermore, not only parameters of the point of care analyzer(s) but also settings of the server (hardware management server) may need to be updated to reflect the change in authorization of the use of the respective point of care analyzer(s) by personnel in the adult's ward authorized to perform diagnostic procedures on adult's samples.

Using known point of care testing POCT systems managed by known servers (hardware management server), the point of care coordinator POCC needs to catalog all analyzer(s) to be relocated, change their corresponding settings in the server (hardware management server) at his workstation. Also, the point of care coordinator POCC needs to physically locate each point of care analyzer(s), change their respective settings to reflect the relocation. This process is not only time-consuming but also error prone. Therefore the point of care coordinator POCC has no convenient and efficient means to replace a non-functional point of care analyzer(s) with a backup analyzer(s) using known point of care testing POCT systems. While this process is feasible in a healthcare facility with a limited number of analyzers, it is a real challenge when a point of care coordinator POCC is responsible for hundreds if not thousands of point of care analyzers.

However, none of the known systems offer convenient workflow(s) to (re)configure point of care analyzers remotely, (re)configuration workflow(s) such as relocation of one or more point of care analyzer(s) or replacement of non-functional point of care analyzer(s) with backup analyzer(s).

According to certain further embodiments of the disclosed point of care testing POCT system 1 respectively methods for configuration thereof, the configuration command includes a relocation command corresponding to a relocation of one or more point of care analyzer(s) 10.1 - 10.n, which is transmitted to the server 50, which updates one or more system parameter(s) in accordance with the relocation of the identified point of care analyzer(s) 10.n - 10.n and retrieves at least one analyzer parameter update in accordance with the relocation of the identified point of care analyzer(s) 10.n - 10.n to be transmitted within the analyzer parameter update to the identified point of care analyzer(s) 10.n - 10.n.

Figure 9 shows a use case diagram of an embodiment of the disclosed system / method for configuration of a point of care testing POCT system 1, illustrating a relocation workflow of a point of care analyzer 10.1 - 10.n. The relocation includes both a physical and/or logical relocation of one or more point of care analyzer(s) 10.1 - 10.n. For example a physical relocation refers to a change of physical location from one healthcare facility/ building/ building floor etc. to another. On the other hand, the logical relocation refers to a change in the reassignment of one or more point of care analyzer(s) 10.1 - 10.n for example between different hospital wards, emergency departments, intensive care units, primary care setting, medical centers, patient homes, a physician's office, a pharmacy or a sites of an emergency.

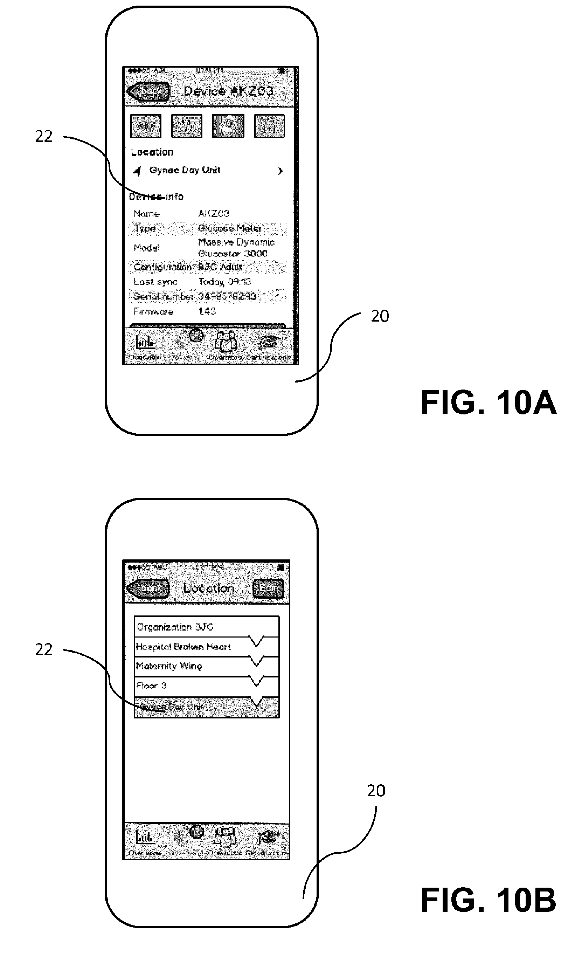

As shown on the use case diagram of Figure 9 and the screenshots of Figures 10A and 10B, the configuration command received by the portable computing device 20 includes a relocation command corresponding to a relocation of one or more point of care analyzer(s) 10.1 - 10.n. This may be in the form of a selection of a physical and/or logical location from a list, a map or manual input of a location or a combination thereof. As shown on Figure 9, according to embodiments of the disclosed system/ method directed towards a relocation of point of care analyzer(s) 10.1 - 10.n, the server 50 is configured to update one or more system parameter(s) in accordance with the relocation of the identified point of care analyzer(s) 10.1 - 10.n and to retrieve at least one analyzer parameter in accordance with the relocation of the identified point of care analyzer(s) 10.1 - 10.n to be transmitted within the analyzer parameter update to the identified point of care analyzer(s) 10.1 - 10.n. For example, upon receiving a relocation command including as new location a children's hospital ward, the server 50 will retrieve all analyzer parameters such as different reference values (as different values apply when analyzing patient samples of children as compared to adults), new workflow definitions (as different locations may use different workflows), new Quality Control QC parameters, etc. (see definition of system/ analyzer parameters above). The analyzer parameters retrieved in this step are referred to as retrieved analyzer parameters.

As a following step, the server 50 transmits the retrieved analyzer parameter(s) to the point of care analyzer(s) 10.1 - 10.n to be relocated (that is the identified point of care analyzer(s) 10.1 - 10.n by the portable computing device 20).

One further aspect of the maintenance operations of a point of care coordinator POCC is to replace a non-functional point of care analyzer(s) with a backup analyzer(s) and/or to relocate a point of care analyzer(s) as needed, these operations including (re)configuration of the respective point of care analyzer(s) and the server (hardware management server) according to the replacement/relocation of analyzer(s).

Using available point of care testing POCT system(s) managed by known hardware management servers, the point of care coordinator POCC has to fetch the non-functional point of care analyzer(s) and the backup analyzer(s) and change their allocation on the server at his workstation, updating all the corresponding settings so that the backup analyzer(s) take the place of the non-functional point of care analyzer(s). Thereafter the point of care coordinator POCC (or an assistant/ nurse etc.) needs to take the (now replaced) point of care analyzer(s) to the location where this is needed. When a point of care coordinator POCC is responsible for hundreds of analyzers possibly at different locations, this is a tedious and time-consuming task, which is especially problematic in an environment where clinical decision making in the emergency department, intensive care units or primary care setting can be dependent on analysis to be performed by the replacement analyzer.

Alternatively the operator (an assistant/ nurse) of the non-functional point of care analyzer(s) could call (or email or notify by other suitable means) the point of care coordinator POCC and provide him with the details of the non-functional point of care analyzer(s) - such as its identifier (e.g. barcode) so that the point of care coordinator POCC can update all the corresponding settings so that the backup analyzer(s) take the place of the non-functional point of care analyzer(s). However, this solution can be also time-consuming and error-prone, especially as often it is only the point of care coordinator POCC - who already travelled onsite - the one to determine that the point of care analyzer(s) is non-functional and needs to be replaced.

Therefore the point of care coordinator POCC has no convenient and efficient means to replace a non-functional point of care analyzer(s) with a backup analyzer(s) using known systems.

According to certain further embodiments of the disclosed point of care testing POCT system 1 respectively methods for configuration thereof, the configuration command includes a replacement command corresponding to a replacement of a first point of care analyzer 10.1 (e.g. a broken analyzer) with a second point of care analyzer 10.2 (e.g. a replacement analyzer). The replacement command is transmitted by the portable computing device 20 to the server 50, which retrieves one or more system parameter(s) corresponding to the first point of care analyzer 10.1, updates therewith respective system parameter(s) corresponding to the second point of care analyzer 10.2 and retrieves one or more analyzer parameter(s) corresponding to the first point of care analyzer 10.1 to be transmitted within the analyzer parameter update to the second point of care analyzer 10.2.

Figure 11 shows a use case diagram of an embodiment of the disclosed system / method for configuration of a point of care testing POCT system 1, illustrating a replacement workflow of a first point of care analyzer 10.1 with a second point of care analyzer 10.2. The replacement of the first point of care analyzer 10.1 may be required for several reasons, including (but not limited to) replacement of a non-functional (broken) or not-fully-functional (partially broken) analyzer with a backup analyzer with identical or at least similar capability(s) of analyzing one or more patient sample(s). The term 'non-functional' or 'not-fully-functional' with reference to a point of care analyzer shall mean that the particular point of care analyzer is at least at that moment not capable to perform or not capable to perform at a required quality/ speed at least one of its functions, such as the analysis of a patient sample. Thus the term 'non-functional' or 'not-fully-functional' do not necessarily mean that the point of care analyzer is completely defective/ broken. Furthermore, the term 'non-functional' or 'not-fully-functional' comprise software- and/or hardware aspects of non-functionality.

Alternatively, an analyzer may be replaced with a different analyzer which is at the time not in use or when the priority of analyzing more patient sample(s) so requires. In a different usage scenario, the replacement workflow of a first point of care analyzer 10.1 with a second point of care analyzer 10.2 is performed within an update/exchange or maintenance of the point of care testing POCT system 1.

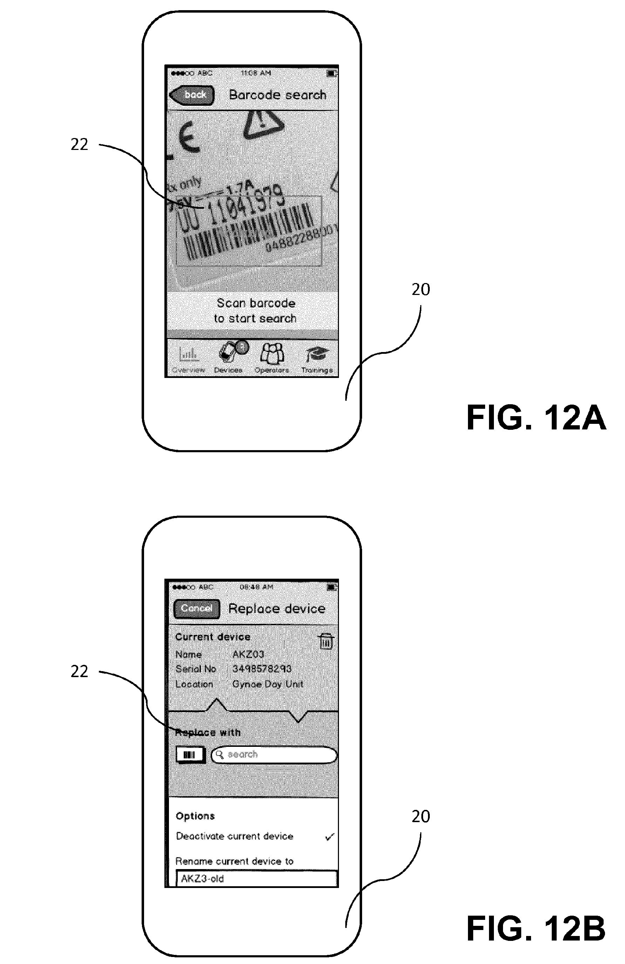

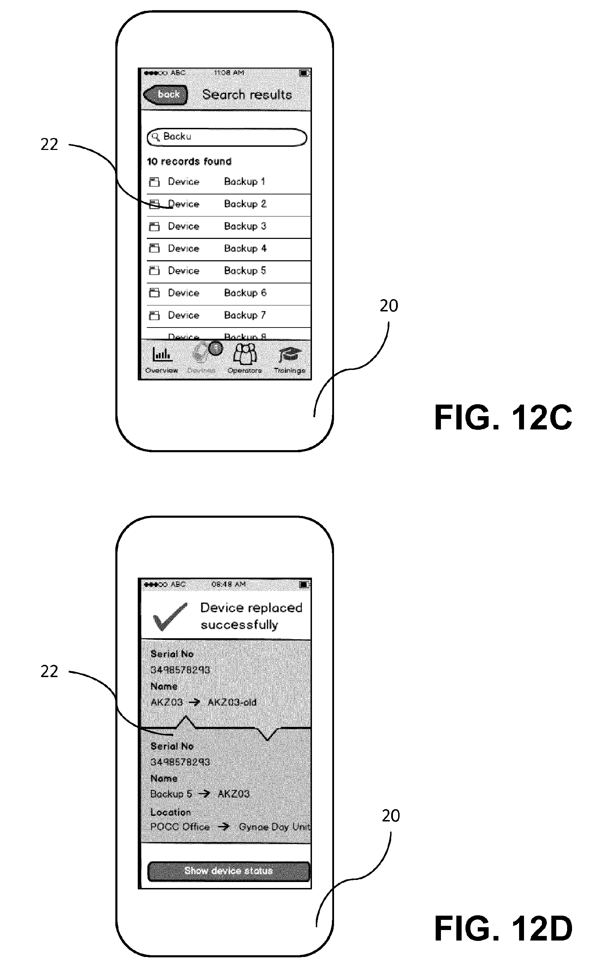

As illustrated on Figure 11, in a step, the portable computing device 20 identifies a first point of care analyzer 10.1 which needs to be replaced and a second point of care analyzer 10.2 on basis of the corresponding analyzer identifier(s). Thereafter the device replacement command is received (as shown on the screenshot of Figure 12B) corresponding to a replacement of the first point of care analyzer 10.1 with the second point of care analyzer 10.2. According to particular embodiments, the replacement command is comprised within the configuration command. Alternatively a separate command may be given by the operator via the user interface 22.

According to alternative embodiments, the replacement command can be received before the second point of care analyzer 10.2 is identified.

After receiving the replacement command and identifying both the first point of care analyzer 10.1 which needs to be replaced and the second point of care analyzer 10.2, the portable computing device 20 generates the configuration request including the replacement command and the analyzer identifiers corresponding to the first point of care analyzer 10.1 and to the second point of care analyzer 10.2.

As shown on Figure 11, according to embodiments of the disclosed system/ method directed towards an analyzer replacement, the server 50 is configured to retrieve one or more system parameter(s) corresponding to the first point of care analyzer 10.1 and update therewith respective system parameter(s) corresponding to the second point of care analyzer 10.2. The system parameters retrieved in this step will be referred to as retrieved system parameters. In other words the system parameters corresponding to the first point of care analyzer 10.1 are copied / moved to update the system parameter(s) for the second point of care analyzer 10.2. After updating the system parameters, the second point of care analyzer 10.2 takes the place of the first point of care analyzer 10.1 in the point of care testing POCT system 1. However, in order for the replacement to be fully completed and for the operator to perceive and be able to use the second point of care analyzer 10.2 as it would be the first point of care analyzer 10.1 (at least functionally- in case the two analyzers are not identical in appearance), analyzer parameter(s) corresponding to the first point of care analyzer 10.1 are retrieved by the server 50 and sent to the second point of care analyzer 10.2 as analyzer parameter update(s). The analyzer parameters retrieved in this step are referred to as retrieved analyzer parameters.

Since the second point of care analyzer 10.2 is configured to receive analyzer update command(s) and to update at least one analyzer parameter according to the corresponding analyzer parameter update, the analyzer parameters of the first point of care analyzer 10.1 are copied/ moved to the second point of care analyzer 10.2.

Therefore embodiments of the disclosed system/ method directed towards an analyzer replacement are particularly advantageous as they provide a simple and efficient workflow solution to replacing a point of care analyzer by a single command given on a portable computing device, wherein the disclosed system/ method takes care of the necessary steps (on the server and the involved point of care analyzers) so that thereafter an operator can use the replacement point of care analyzer providing the same functionalities and/or same setup/configuration and/or user rights, etc. (see the non-exhaustive list of system parameter and/or analyzer parameter above) as the point of care POC device which had to be replaced. This ensures a very positive user experience as service/ maintenance and/or analyzer update activities can be made transparent to the operator and downtime of a particular type of point of care analyzer can be eliminated or at least minimized.

According to embodiments of the disclosed system / method, the server 50 can be configured to flag the first point of care analyzer 10.1 as inactive after updating the system parameter(s) corresponding to the second point of care analyzer 10.2. The point of care coordinator POCC can then inspect point of care analyzers flagged or have them inspected by a technician. After repair (hardware and/or software) the inactive flag of the respective point of care analyzers are removed and become available for use again, be it immediately or as a backup analyzer for use as replacement.

A further challenge in the management of point of care testing POCT is posed by the configuration management of operator training and of the corresponding training certification(s). Traditionally the management of operator training / certification(s) implies that training / certification data has to be entered centrally often even manually in a paper based approach. An obvious consequence of this type of training / certification management is that the probability of an error or delays is higher. This situation can have a high impact on efficiency and can even create some issues regarding the speed of patient care.

However, the certification management of prior art solutions is performed centrally. This approach has several disadvantages, especially in dynamic point of care POC environments with a high number of operators requiring training (in the thousands to tens of thousands across multiple healthcare facilities), a high turnover rate of operators who may be transferred between different departments and operators with diverse educational backgrounds. Thus, in comparison with a more static environment where trainings and examinations can be planned in advance and corresponding certificates can be managed centrally, in a dynamic environment there is a need to be able to perform the management of operator training / certification / status in an ad-hoc manner and if needed both on-site - namely at or near the particular point of care analyzer, the patient and/or the healthcare staff (the operator) - or off-site - which may be necessary in cases when it is not feasible for the point of care coordinator POCC to relocate (for time/distance/resource constraints).

According to certain further embodiments disclosed, the configuration of the point of care testing POCT system 1, in order to manage operator certifications, is performed as follows:
- one or more system certification(s) 30.1-30.m, each corresponding to one or more point of care analyzer(s) 10.1 - 10.n are stored on the server 50;
- operators of the point of care testing POCT system 1 are provided with corresponding operator identifier(s);
- one or more certification(s) 30.1-30.m are selected via the user interface 22 of the portable computing device 20;
- one or more operator(s) are identified using the operator identifier(s);
- a certification configuration command is given via the user interface 22 of the portable computing device 20;
- a certification configuration request is generated by the portable computing device 20, the certification configuration request comprising:
   - the one or more operator identifier(s) corresponding to the identified operator(s); and
   - data identifying the selected system certification(s) 30.1-30.m;
- the server receives the configuration request;
- the server 50 updates the selected system certification(s) 30.1-30.m according to the certification configuration request;
- the server 50 transmits an analyzer certification update for each of the selected system certification(s) 30.1-30.m to the corresponding point of care analyzer(s) 10.1 - 10.n;
- the one or more point of care analyzer(s) 10.1 - 10.n receive the analyzer certification update;
- the one or more point of care analyzer(s) 10.1 - 10.n update their respective analyzer certification according to the analyzer certification update.

Such embodiments of the disclosed system / method are particularly advantageous for allowing a decentralized control of analyzer certifications and permitting that a point of care coordinator POCC easily adds / deletes/ updates operators (their status) to the system. Status update(s) can be related to the completion of training / successful examination or an operator access update. This way such management steps can be done immediately via a portable computing device in an efficient and secure manner.

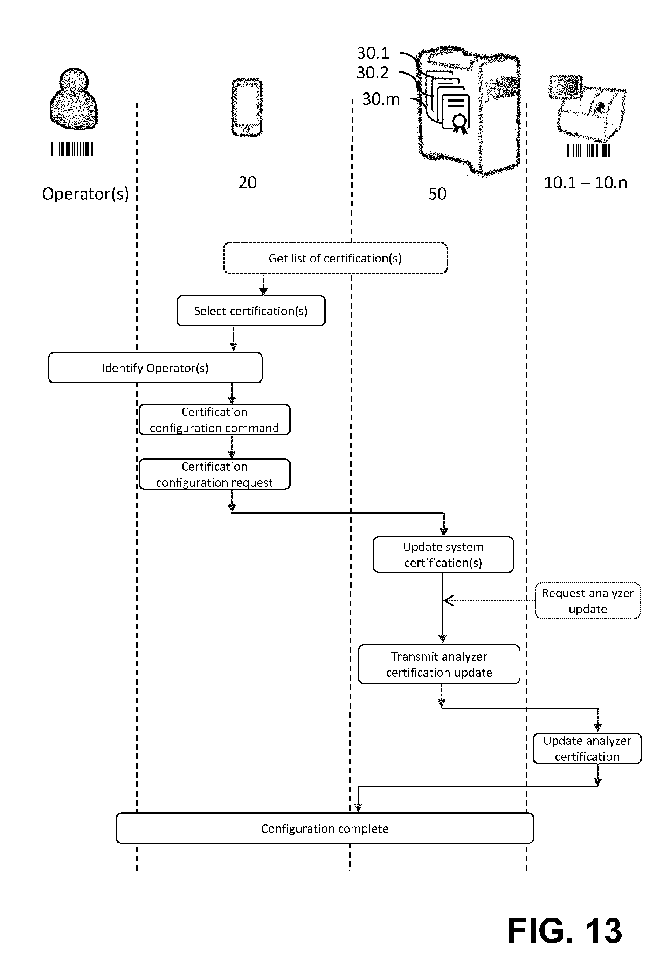

Figure 13 shows a use case diagram of further embodiments of the disclosed system/ method for configuration of a point of care testing POCT system, illustrating a certification configuration corresponding to one or more operator(s) and one or more certification(s).

According to embodiments of the disclosed system/ method, the server 50 can be further configured for storing one or more system certification(s) 30.1-30.m, each corresponding to one or more point of care analyzer(s) 10.1 - 10.n. As shown on Figure 10A, according to particular embodiments, the server 50 can be configured to store a system certification 30.1-30.m for each type / class of point of care analyzers. For this, analyzers of the same type/ class (with similar functionality of analyzing patient sample(s)) are grouped and a system certification 30.1-30.m can be stored for each type/ class, each system certification 30.1-30.m storing the operator identifier(s) of each operator certified to operate one or more point of care analyzer(s) 10.1 - 10.n of the type/ class. Alternatively, the server 50 can be configured to store a system certification 30.1-30.m for each of the one or more point of care analyzer(s) 10.1 - 10.n.

In a step (of certain embodiments - the step being shown with dotted lines) - the portable computing device 20 can be configured to request the one or more system certifications 30.1-30.m and/or a list of the one or more system certifications 30.1-30.m from the server 50, while the server 50 can be configured to transmit the one or more system certifications 30.1-30.m and/or a list of the one or more system certifications 30.1-30.m to the portable computing device 20.

Thereafter one or more of the system certifications 30.1-30.m are selected via the user interface 22 of the portable computing device 20 as shown on the screenshot of Figure 10A, these are referred to as the selected system certifications 30.1-30.m.

For identifying operator(s) of the one or more point of care analyzer(s) 10.1 - 10.n of the point of care testing POCT system 1, operator(s) are provided with operator identifier(s), each operator identifier uniquely identifying the respective operators. Correspondingly, the portable computing device 20 is configured to identify one or more operator(s) of the point of care testing POCT system 1 using one or more operator identifier(s). The one or more operator(s) identified by the portable computing device 20 are referred to as the identified operator(s).

According to embodiments of the disclosed system/ method, the one or more operator identifier(s) are identifier tags, such as a barcode and/or an RFID tag and/or an alphanumeric identifier. Correspondingly the portable computing device 20 includes an identifier reader such as a barcode reader and/or an RFID reader to read the identifier tag and/or input means (such as a keyboard or input field on a screen) for inputting an alphanumeric identifier of the one or more operator identifier(s). In addition or alternatively, a camera device may be provided to identify the operators based on the operator identifier(s). In addition or alternatively biometric identification of the operator(s) may be used.

After one or more operator(s) have been identified and one or more certifications 30.1-30.m have been selected, a certification configuration command is received via the user interface 22 of the portable computing device 20. According to embodiments of the user interface 22, the certification configuration command may be a push of a button (physical or screen button) a voice command, a selection in a menu, etc. The certification configuration command may be any form of input from an operator to initiate a configuration workflow (process).

Initiated by the certification configuration command, the portable computing device 20 generates a certification configuration request comprising:
- the one or more operator identifier(s) corresponding to the identified operator(s); and
- data identifying the selected system certification(s) 30.1-30.m.

According to embodiments of the disclosed system/ method, the certification configuration request transmitted by the portable computing device 20 to the server 50 includes one or more of the following:
- addition of an operator status corresponding to the identified operator(s); and/or
- removal of an operator status corresponding to the identified operator(s); and/or
- update of an operator status corresponding to the identified operator(s).

After generating it (optionally after confirmation from the operator), the portable computing device 20 transmits the certification configuration request to the server 50. Therefore the certification configuration request can be described as a sort of translation of the certification configuration command from the operator into a request signal to the server 50.

After receiving the certification configuration request - via the communication network 70 - the server 50:
- updates the selected system certification(s) 30.1-30.m according to the certification configuration request; and
- transmits an analyzer certification update for each of the selected system certification(s) 30.1-30.m to the corresponding point of care analyzer(s) 10.1 - 10.n.

The above steps by the server 50 shall now be described in greater detail. On one hand the server 50 is configured to update the selected system certification(s) 30.1-30.m within the server 50 according to the certification configuration request. On the other hand, in order to ensure that the certification configuration command (as a complete workflow) is implemented not only on the server 50 but across the point of care testing POCT system 1, the server 50 is configured to transmit an analyzer update for each of the selected system certification(s) 30.1-30.m to the corresponding point of care analyzer(s) 10.1 - 10.n.

According to embodiments of the disclosed system/ method, the analyzer certification update transmitted by the server 50 to the corresponding point of care analyzer(s) 10.1
- 10.n includes one or more of the following:
- one or more operator identifier(s) corresponding to the identified operator(s) to be granted access the respective point of care analyzer 10.1 - 10.n; and/or
- one or more operator identifier(s) corresponding to the identified operator(s) to be denied access to the respective point of care analyzer 10.1 - 10.n; and/or
- one or more operator identifier(s) corresponding to the identified operator(s) to be granted limited access to the respective point of care analyzer 10.1 - 10.n.

According to embodiments of the disclosed system/ method, the steps of:
- the server 50 transmitting an analyzer certification update for each of the selected system certification(s) 30.1-30.m to the corresponding point of care analyzer(s) 10.1 - 10.n;
- the one or more point of care analyzer(s) 10.1 - 10.n receiving the analyzer certification update;
- the one or more point of care analyzer(s) 10.1 - 10.n updating their respective analyzer certification according to the analyzer certification update
are initiated by one or more point of care analyzer(s) 10.1 - 10.n requesting an update of the corresponding system certification(s) 30.1-30.m. Correspondingly, the one or more point of care analyzer(s) 10.1 - 10.n are configured to request analyzer certification update(s) on occurrence(s) of certain event(s) and/or at regular intervals. According to embodiments of the disclosed system/ method, the one or more point of care analyzer(s) 10.1 - 10.n are configured to request an analyzer certification update upon a login by an operator and/or upon startup and/or upon shutdown and/or upon docking (into a docking station) of the respective point of care analyzer 10.1 - 10.n. This is shown on the use case diagrams with dotted lines.

To summarize the analyzer certification update(s) - according to embodiment(s) of the disclosed system/ method - the analyzer certification update(s) may be implemented using "server push" technology (that is server initiated) and/or a "client pull" technology (client - in this case point of care analyzer initiated). The server push implementation of analyzer certification update(s) is advantageous for point of care analyzers 10.1 - 10.n which are continuously communicating with the server, while the client pull implementation of analyzer certification update(s) is advantageous for point of care analyzers 10.1 - 10.n which communicate with the server only on an event basis, such as periodically and/or upon a login by an operator and/or upon startup and/or upon shutdown of the respective point of care analyzer 10.1 - 10.n. Also, according to embodiments of the disclosed system/ method, the point of care analyzers 10.1 - 10.n request an analyzer certification update from the server 50 upon expiry of a validity of the respective system certification(s) 30.1-30.m.

According to particular embodiments of the disclosed system/ method, as shown on the screenshot of Figure 10B, receiving the certification configuration command via the user interface 22 of the portable computing device 20 includes a selection of one or more certification criteria via the user interface 22. Certification criteria includes (but is not limited to) one or more of the following:
- Classroom training;
- Hands-on training;
- Training on patient sample analysis;
- Training on calibration and/or quality control.

In embodiments of the disclosed system/ method according to which one or more certification criteria corresponding to one or more system certification(s) 30.1-30.m are defined, the one or more point of care analyzer(s) 10.1 - 10.n are configured to control access of the identified operator(s) to the respective point of care analyzer 10.1 - 10.n according to the one or more certification criteria of the selected system certification(s) 30.1-30.m. Such control of access includes allowing an operator to use only certain functions of the point of care analyzer 10.1 - 10.n. For example an operator who has the certification criterion "Training on patient sample measurement" but not the certification criterion "Training on calibration and/or quality control" may only perform patient sample analysis with the respective point of care analyzer(s) 10.1 - 10.n but not calibration and quality control.

According to embodiments of the disclosed system/ method, the server 50 can be further configured to:
- mark a list of authorized operators for each of the point of care analyzer(s) 10.1 - 10.n corresponding to the selected system certification(s) 30.1-30.m as invalid; and/or
- update a list of authorized operators for each of the point of care analyzer(s) 10.1-10.n corresponding to the selected system certification(s) 30.1-30.m according to the analyzer certification update.

In such embodiment(s) the analyzer certification update includes a list of authorized operators for the respective point of care analyzer(s) 10.1 - 10.n.

According to embodiments of the disclosed system/ method, the point of care analyzer(s) 10.1 - 10.n are configured to perform one or more of the following:
- blood glucose testing;
- coagulation testing;
- blood gas or electrolytes analysis;
- urinalysis;
- cardiac markers analysis;
- hemoglobin diagnostics;
- infectious disease testing
- cholesterol screening;
- nucleic acid testing NAT.

According to embodiments of the disclosed system / method, the portable computing device 20 can be one of the following:
- a mobile phone, in particular a smartphone;
- a tablet computer;
- a laptop computer;
- a dedicated PDA device.

According to embodiments of the disclosed system/ method, the server 50 can be configured to:
- retrieve analytical data from the one or more point of care analyzer(s) 10.1 - 10.n such as data representing the measurement of patient health parameter(s);
- update program data of the one or more point of care analyzer(s) 10.1 - 10.n such as a software update.

It will be understood that not all steps of the methods herein disclosed are necessarily carried out in the listed/ described order. In particular, a configuration command may be received by the portable computing device before identifying point of care analyzer(s); operators may be identified before selecting a certification; analyzer update command(s) may be transmitted by the server to the point of care analyzer(s) before updating system parameter(s) or a first point of care analyzer may be flagged as inactive before the second (replacement) point of care analyzer is updated with the analyzer parameters of the first point of care analyzer.

**REFERENCE SIGNS LIST**

| | |
|---|---|
| point of care testing POCT system | 1 |
| point of care analyzer(s) | 10.1 - 10.n |
| portable computing device | 20 |
| user interface (of the portable computing device) | 22 |
| system certification(s) | 30.1-30.m |
| server | 50 |
| communication network | 70 |
| point of care POC communication network area | 71 |
| remote configuration network area | 72 |
| portable device to analyzer network area | 73 |
| first communication subnetwork | 71.1 |
| second communication subnetwork | 71.2 |
| hardware management server | 52 |
| first hardware management subserver | 52.1 |
| second hardware management subserver | 52.2 |
| server interface | 53 |
| first subserver interface | 53.1 |
| second subserver interface | 53.2 |
| remote configuration server | 56 |
| authentication and authorization unit | 57 |
| adapter unit | 58 |
| directory server | 59 |
| management console | 60 |
| Laboratory Information System LIS / Hospital Information system HIS | 200 |

## Claims

1. A point of care testing POCT system (1) for analyzing biological samples comprising:
- one or more point of care analyzer(s) (10.1 - 10.n) for analyzing one or more patient sample(s);
- a portable computing device (20);
- a hardware management server (52) for storing system parameter(s) corresponding to the one or more point of care analyzer(s) (10.1 - 10.n);
- a remote configuration server (56);
- a communication network (70) configured to communicatively connect:
- the point of care analyzer(s) (10.1 - 10.n) with the hardware management server (52);
- the portable computing device (20) with the remote configuration server (56); and
- the remote configuration server (56) with the hardware management server (52),
wherein the remote configuration server (56) is configured to:
- authenticate and authorize the portable computing device (20);
- receive a configuration request from the portable computing device (20);
- transmit the configuration request to the hardware management server (52);
wherein the hardware management server (52) is configured to receive and process the configuration request in order to:
- update at least one system parameter according to the configuration request; and
- transmit an analyzer update command comprising at least one analyzer parameter update to one or more point of care analyzer(s) (10.1 - 10.n) according to the configuration request; and
wherein the one or more point of care analyzer(s) (10.1 - 10.n) are configured to receive the analyzer update command and to update at least one analyzer parameter (15) according to the respective analyzer parameter update.

2. A point of care testing POCT system (1) for analyzing biological samples according to claim 1, wherein:
- the hardware management server (52) is configured to expose a hardware management server interface (53) for communication with the remote configuration server (56);
- the remote configuration server (56) comprises:
- an authentication and authorization unit (57) to authenticate and authorize the portable computing device (20) and/or a point of care coordinator POCC via the portable computing device (20) with the remote configuration server (56);
- an adapter unit (58) configured to adapt the configuration request to the hardware management server interface (53).

3. A point of care testing POCT system (1) for analyzing biological samples according to claim 1 or 2, wherein the hardware management server (52) is further configured to perform one or more of the following:
- retrieve analytical data from the point of care analyzer(s) (10.1 - 10.n);
- update program data of the point of care analyzer(s) (10.1 - 10.n);
- transfer of authentication data update(s) and/or certification update data of the point of care analyzer(s) (10.1 - 10.n).

4. A point of care testing POCT system (1) for analyzing biological samples according to one of the claims 1 to 3, wherein the hardware management server (52) is communicatively connected to one or more of a Laboratory Information System LIS and a Hospital Information system HIS (200).

5. A point of care testing POCT system (1) for analyzing biological samples according to one of the claims 1 to 4, wherein the communication network (70) comprises:
- a point of care POC communication network area (71) for communicatively connecting the point of care analyzer(s) (10.1 - 10.n) with the hardware management server (52);
- a remote configuration network area (72) for communicatively connecting the portable computing device (20) with the remote configuration server (56).

6. A point of care testing POCT system (1) for analyzing biological samples according to one of the claims 1 to 5, further comprising:
- a first hardware management subserver (52.1) communicatively connected to a first group of one or more point of care analyzer(s) (10.1 - 10.n); and
- a second hardware management subserver (52.2) communicatively connected to a second group of one or more point of care analyzer(s) (10.1 - 10.n);
wherein:
- the first hardware management subserver (52.1) is configured to transmit the analyzer update command(s) to the one or more point of care analyzer(s) (10.1
- 10.n) of the first group; and
- the second hardware management subserver (52.2) is configured to transmit the analyzer update command(s) to the one or more point of care analyzer(s) (10.1 - 10.n) of the second group.

7. A point of care testing POCT system (1) for analyzing biological samples according to claims 6, wherein:
- the first hardware management subserver(s) (52.1) is configured to expose a first subserver interface (53.1) for communication with the remote configuration server (56);
- the second hardware management subserver(s) (52.2) is configured to expose a second subserver interface (53.2) for communication with the remote configuration server (56);
- the adapter unit (58) of the remote configuration server (56) is configured to adapt the configuration request corresponding to one or more point of care analyzer(s) (10.1 - 10.n) of the first group, in order to accommodate the first subserver interface (53.1);
- the adapter unit (58) of the remote configuration server (56) is configured to adapt the configuration request corresponding to one or more point of care analyzer(s) (10.1 - 10.n) of the second group, in order to accommodate the second subserver interface (53.2).

8. A point of care testing POCT system (1) for analyzing biological samples according to one of the claims 2 to 7, further comprising a directory server (59) configured to store authentication and authorization data and configured to allow sharing of the authentication and authorization data between the directory server (59) and one or more of the hardware management server (52), the first hardware management subserver (52.1), the second hardware management subserver (52.2) for authenticating and authorizing the one or more point of care analyzer(s) (10.1 - 10.n) and operator(s) of the one or more point of care analyzer(s) (10.1 - 10.n).

9. A point of care testing POCT system (1) for analyzing biological samples according to claim 8, wherein the authentication and authorization unit (57) of the remote configuration server (56) and the directory server (59) are configured and communicatively connected in order to allow exchange of authentication and authorization data.

10. Method for configuration of a point of care testing POCT system (1) for analyzing biological samples, comprising the steps of:
- providing one or more point of care analyzer(s) (10.1 - 10.n) for analyzing one or more patient sample(s);
- providing a portable computing device (20);
- providing a hardware management server (52) configured for storing system parameter(s) corresponding to the one or more point of care analyzer(s) (10.1 - 10.n);
- providing a remote configuration server (56);
- communicatively connecting:
- the point of care analyzer(s) (10.1 - 10.n) with the hardware management server (52);
- the portable computing device (20) with the remote configuration server (56); and
- the remote configuration server (56) with the hardware management server (52),
via a communication network (70);
- the remote configuration server (56) authenticating and authorizing the portable computing device (20);
- the remote configuration server (56) receiving a configuration request from the portable computing device (20);
- the remote configuration server (56) transmitting the configuration request to the hardware management server (52);
- the server (50) receiving and processing of the configuration request, the processing comprising:
- updating at least one system parameter according to the configuration request; and
- transmitting an analyzer update command comprising at least one analyzer parameter update to one or more point of care analyzer(s) (10.1 - 10.n) according to the configuration request;
- the one or more point of care analyzer(s) (10.1 - 10.n) receiving the analyzer update command and updating at least one analyzer parameter (15) according to the respective analyzer parameter update.

11. Method for configuration of a point of care testing POCT system (1) for analyzing biological samples according to claim 10, further comprising the steps of:
- the hardware management server (52) exposing a hardware management server interface (53) for communication with the remote configuration server (56);
- providing an authentication and authorization unit (57) of the remote configuration server (56);
- the authentication and authorization unit (57) authenticating and authorizing the portable computing device (20) and/or a point of care coordinator POCC via the portable computing device (20) with the remote configuration server (56);
- providing an adapter unit (58) of the remote configuration server (56); and
- the adapter unit (58) adapting of the configuration request to the hardware management server interface (53).

12. Method for configuration of a point of care testing POCT system (1) for analyzing biological samples according to claim 11, wherein the step of adapting the configuration request received from the portable computing device (20) comprising one or more of following steps:
- formatting and/or reformatting the configuration request to a format compatible with the hardware management server interface (53)
- adding authentication and/or authorization data to the configuration request to authenticate and authorize the remote configuration server (56) with the hardware management server (52).

13. Method for configuration of a point of care testing POCT system (1) for analyzing biological samples according to one of the claims 10 to 12, further comprising the steps of:
- communicatively connecting a first group of one or more point of care analyzer(s) (10.1 - 10.n) with a first hardware management subserver(s) (52.1);
- communicatively connecting a second group of one or more point of care analyzer(s) (10.1 - 10.n) with a second hardware management subserver(s) (52.2);
- the first hardware management subserver (52.1) transmitting the analyzer update command(s) to the one or more point of care analyzer(s) (10.1 - 10.n) of the first group; and
- the second hardware management subserver (52.2) transmitting the analyzer update command(s) to the one or more point of care analyzer(s) (10.1 - 10.n) of the second group.

14. Method for configuration of a point of care testing POCT system (1) for analyzing biological samples according to claim 13, further comprising the steps of:
- the first hardware management subserver (52.1) exposing a first subserver interface(53.1) for communication with the remote configuration server (56);
- the second hardware management subserver (52.2) exposing a second subserver interface(53.2) for communication with the remote configuration server (56);
- the adapter unit (58) adapting the configuration request corresponding to one or more point of care analyzer(s) (10.1 - 10.n) to the first subserver interface (53.1); and
- the adapter unit (58) adapting the configuration request corresponding to one or more point of care analyzer(s) (10.1 - 10.n) of the second group to the second subserver interface (53.2).

15. Method for configuration of a point of care testing POCT system (1) for analyzing biological samples according to one of the claims 10 to 14, further comprising the steps of:
- providing a directory server (59);
- storing authentication and authorization data on the directory server (59); and
- sharing of authentication and authorization data between the directory server (59) and the hardware management server (52) and/or the first hardware management subserver (52.1) and/or the second hardware management subserver (52.2) for authenticating and authorizing the one or more point of care analyzer(s) (10.1 - 10.n) and/or operator(s) of the one or more point of care analyzer(s) (10.1 - 10.n).
